# EUROPÄISCHE PATENTSCHRIFT

(11) **EP 3 063 122 B1**
(45) Veröffentlichungstag und Bekanntmachung des Hinweises auf die Patenterteilung: **28.08.2019**
(21) Anmeldenummer: 14793073.9
(22) Anmeldetag: 30.10.2014
(51) Int. Cl.: C07C 67/08, C07C 69/80, C07C 69/82, C08K 5/12

(54) **VERFAHREN ZUR HERSTELLUNG VON CARBONSÄUREESTERN UND DEREN VERWENDUNG ALS WEICHMACHER**
METHOD FOR PRODUCING CARBOXYLIC ACID ESTERS AND THE USE THEREOF AS PLASTICIZERS
PROCÉDÉ DE PRODUCTION D'ESTERS D'ACIDE CARBOXYLIQUE ET UTILISATION DESDITS D'ESTERS D'ACIDE CARBOXYLIQUE COMME PLASTIFIANT

(30) Priorität: 31.10.2013 EP 13191071; 11.08.2014 EP 14180491
(43) Veröffentlichungstag der Anmeldung: 07.09.2016
(73) Patentinhaber: BASF SE, 67056 Ludwigshafen am Rhein (DE)
(72) Erfinder: KALLER, Martin, 68163 Mannheim (DE); KOCH, Michael, 67346 Speyer (DE); BREITSCHEIDEL, Boris, 67165 Waldsee (DE)
(86) Internationale Anmeldenummer: PCT/EP2014/073272
(87) Internationale Veröffentlichungsnummer: WO 2015/063189

(56) Entgegenhaltungen:
- WO-A1-00/78702
- WO-A1-2010/076192
- JP-A- 2003 160 535
- US-A- 4 241 216
- David F. Cadogan ET AL: "Plasticizers" In: "Ullmann's Encyclopedia of Industrial Chemistry", 15. Juni 2000 (2000-06-15), Wiley-VCH Verlag GmbH & Co. KGaA, Weinheim, Germany, XP055167665, ISBN: 978-3-52-730673-2 DOI: 10.1002/14356007.a20_439, section 2.2 "Phthalate Esters" (page 601)

## Beschreibung

### HINTERGRUND DER ERFINDUNG

Die vorliegende Erfindung betrifft ein Verfahren zur Herstellung von Carbonsäureestern durch Umsetzung von Carbonsäuren und/oder Carbonsäureanhydriden mit wenigstens einem Alkohol, ausgewählt unter Alkanolen mit mindestens 5 Kohlenstoffatomen, Cycloalkanolen und Alkoxyalkanolen, in Gegenwart von Methansulfonsäure, wobei die Methansulfonsäure einen Sulfatgehalt von höchstens 50 ppm aufweist. Die so erhaltenen Carbonsäureester können als Weichmacher oder in einer Weichmacherzusammensetzung für thermoplastische Polymere und Elastomere verwendet werden.

### STAND DER TECHNIK

Ester aliphatischer und aromatischer Carbonsäuren mit Alkanolen, Cycloalkanolen oder Alkoxyalkanolen haben in der Technik vielfältig Verwendung gefunden. Sie finden beispielsweise weite Anwendung in Lackharzen und als Bestandteile von Anstrichmitteln, wobei speziell die Ester der Essigsäure, Phthalsäure, Trimellitsäure, Terephthalsäure, Adipinsäure, Sebacinsäure oder Maleinsäure eingesetzt werden. Sie eignen sich desweiteren speziell als Weichmacher oder als Komponente einer Weichmacherzusammensetzung für thermoplastische Polymere und Elastomere.

Weichmacher werden einer Vielzahl von Kunststoffen zur Erzielung der gewünschten Verarbeitungs- bzw. Anwendungseigenschaften zugesetzt, um diese weicher, flexibler und/oder dehnbarer zu machen. Im Allgemeinen dient der Einsatz von Weichmachern dazu, den thermoplastischen Bereich von Kunststoffen zu niedrigeren Temperaturen hin zu verschieben, um im Bereich niedriger Verarbeitungs- und Einsatztemperaturen die gewünschten elastischen Eigenschaften zu erhalten. Wichtige thermoplastische Polymere in denen üblicherweise Weichmacher Anwendung finden sind neben Polyvinylchlorid (PVC) beispielsweise Polyvinylbutyral (PVB), Homo- und Copolymere von Styrol, Polyacrylate, Polysulfide oder thermoplastische Polyurethane (PU). Aufgrund ihrer guten Verträglichkeit mit PVC und weiteren Polymeren und ihrer vorteilhaften anwendungstechnischen Eigenschaften wurden in der Vergangenheit vielfach Phthalsäurediester mit Alkoholen unterschiedlicher chemischer Struktur als Weichmacher eingesetzt, wie z. B. Diethylhexylphthalat (DEHP). Da diese jedoch toxikologisch nicht unbedenklich sind, wurden sie in letzter Zeit speziell für sensible Anwendungsbereiche wie Kinderspielzeug, Lebensmittelverpackungen oder medizinische Artikel durch andere Weichmacher ersetzt. Hier sind insbesondere die Ester weiterer aromatischer Carbonsäuren, wie der Terephthalsäure, Trimellitsäure und Benzoesäure von Bedeutung.

Es ist bekannt, Carbonsäureester durch Umsetzung von Carbonsäuren mit Alkoholen herzustellen. Diese Reaktion kann autokatalytisch oder katalytisch, beispielsweise durch Brönsted- oder Lewissäuren, durchgeführt werden. Derartige Verfahren sind in Lorz et al., Phthalic Acid and Derivatives, Ullmann's Encyclopedia of Industrial Chemistry, 2007, Seiten 131 - 180 (DOI: 10.1002/14356007.a20_181.pub2) beschrieben. Bei der autokatalytischen Veresterung liegen die Reaktionstemperaturen üblicherweise bei > 200 °C. Dennoch werden in der Regel nur Teilumsätze erreicht, so dass ein Recycling der zurückbleibenden Carbonsäure zwingend erforderlich ist.

Unabhängig von der Art der Katalyse entsteht immer ein temperaturabhängiges Gleichgewicht zwischen den Einsatzstoffen (Carbonsäure und Alkohol) und den Produkten (Ester und Wasser). Die Umsetzung von inneren Carbonsäureanhydriden mit Alkoholen verläuft in zwei Schritten: Die Alkoholyse des Anhydrids zum Monoester verläuft in der Regel rasch und vollständig. Die weitere Umsetzung des Monoesters zum Diester unter Bildung von Reaktionswasser ist reversibel und verläuft langsam. Dieser zweite Schritt ist der geschwindigkeitsbestimmende Schritt der Reaktion. Um das Gleichgewicht zu Gunsten des Esters (bzw. des Vollesters bei mehrbasigen Säuren) zu verschieben, wird in der Regel ein Schleppmittel eingesetzt, mit dessen Hilfe das Reaktionswasser aus dem Ansatz entfernt wird. Wenn einer der Einsatzstoffe (Alkohol oder Carbonsäure) niedriger siedet als der gebildete Ester und mit Wasser eine Mischungslücke bildet, kann ein Edukt als Schleppmittel verwendet und nach Abtrennung von Wasser wieder in den Ansatz zurückgeführt werden. Bei der Veresterung von höheren aliphatischen Carbonsäuren, aromatischen Carbonsäuren oder von zwei- oder mehrbasigen Carbonsäuren ist in der Regel der eingesetzte Alkohol das Schleppmittel.

Typische Veresterungskatalysatoren zur Herstellung von als Weichmachern geeigneten Carbonsäureestern sind Tetraalkyltitanate.

US 7,799,942 beispielsweise offenbart ein Verfahren zur Herstellung von Diestern der Terephthalsäure, wie beispielsweise Bis(2-ethylhexyl)terephthalat (DOTP), bei dem man Terephthalsäure und einen C₆-C₁₀-Alkohol einer Veresterung in Gegenwart eines Tetraalkyltitanats als Katalysator unterzieht, wobei das während der Veresterung entstehende Wasser und ein Teil des Alkohols durch Hindurchleiten eines Inertgases durch die Reaktionszone oder mit Hilfe einer Destillationskolonne entfernt werden.

Der Einsatz von Tetraalkyltitanaten als Katalysatoren ist mit mehreren Nachteilen verbunden. So wird das Reaktionsgemisch zur Entfernung des Katalysators mit einer Base, z. B. wässriger NaOH, versetzt und die resultierenden Hydrolyseprodukte werden abfiltriert. Diese Abtrennung ist zeitaufwändig, so dass nur geringe Raum-Zeit-Ausbeuten erzielt werden. In der Regel ist eine weitere Aufarbeitung des Reaktionsgemisches erforderlich, z. B. eine Destillation zur Entfernung überschüssigen Alkohols und/oder eine Behandlung mit Aktivkohle zur Erzielung akzeptabler Farbzahlen.

Des Weiteren ist im Stand der Technik auch der Einsatz von Mineralsäuren und starken organischen Säuren, wie Methansulfonsäure und p-Toluolsulfonsäure, als Katalysatoren zur Herstellung von Carbonsäureestern beschrieben. In Lorz et al., Phthalic Acid and Derivatives, Ullmann's Encyclopedia of Industrial Chemistry, 2007, Seiten 131 - 180 (DOI: 10.1002/14356007.a20_181.pub2) wird jedoch gelehrt, dass Brönstedsaure Katalysatoren nur bis zu einer Temperatur von 165 °C eingesetzt werden können, da ansonsten störende Nebenreaktionen auftreten, die unter Anderem zur Bildung von Olefinen durch Wassereliminierung aus den eingesetzten Alkoholen und zur Bildung von stark gefärbten Nebenprodukten führen können.

Die WO 2010/076192 beschreibt ein Verfahren zur Herstellung von Carbonsäureestern durch Umsetzung einer Carbonsäure oder eines Carbonsäureanhydrids oder eines Gemisches davon mit einem Alkohol in Gegenwart eines Veresterungskatalysators, bei dem das bei der Veresterung entstehende Reaktionswasser als Alkohol-Wasser-Azeotrop mit dem Brüden abdestilliert wird. Der Brüden wird zumindest teilweise kondensiert, einer Phasentrennung unterzogen und die organische Phase zumindest teilweise in das Reaktionssystem zurückgeführt, wobei aus der zurückzuführenden Phase Komponenten abgetrennt werden, die niedriger sieden als der zur Veresterung eingesetzte Alkohol. Als geeignete Veresterungskatalysator werden verschiedenste amphiphile Metallkatalysatoren sowie Mineralsäuren und organische Säuren genannt. Konkret beschrieben wird die kontinuierliche Herstellung von Diisononylphthalat und Dipropylheptylphthalat bei dem als Veresterungskatalysator eine Mischung aus Isopropyl- und n-Butyltitanat eingesetzt wird.

Die JP 62267341 offenbart ein Verfahren zur Herstellung von Carbonsäureestern zur Verwendung als Weichmacher, erhältlich durch Umsetzung einer Carbonsäure mit einem Alkohol in Gegenwart einer Sulfonsäure als Veresterungskatalysator. Das Veresterungsrohprodukt wird dabei einer Reinigung durch Zugabe einer Base, z. B. CaO oder MgO, und eines festen Adsorbens, wie beispielsweise Aktivkohle, Kieselgur oder aktivierte Bleicherde, zur Verringerung der Säurezahl bzw. der Farbzahl unterzogen.

Die JP 1994157407 offenbart ein Verfahren zur Herstellung von Carbonsäureestern durch Umsetzung einer Carbonsäure mit einem Alkohol in Gegenwart von Methansulfonsäure als Veresterungskatalysator bei einer niedrigen Reaktionstemperatur von 80 bis 120 °C, wobei das Reaktionswasser unter Verwendung einer wasserpermeablen Membran selektiv aus der Reaktionsmischung abgetrennt wird. Als konkretes Ausführungsbeispiel wird die Herstellung von Bis(2-ethylhexyl)-phthalat beschrieben.

JP 1994122652 beschreibt ein Verfahren zur Herstellung von Carbonsäureestern durch Umsetzung einer Carbonsäure oder eines Carbonsäureanhydrids mit einem Alkohol in Gegenwart einer Sulfonsäure als Veresterungskatalysator und eines Zeoliths zur Bindung des während der Veresterung gebildeten Reaktionswassers. Konkret wird die Herstellung von Bis(2-ethylhexyl)-phthalat aus Phthalsäureanhydrid und 2-Ethylhexanol bei einer Reaktionstemperatur von 100 °C in Gegenwart von Methansulfonsäure als Katalysator beschrieben, bei der Zeolith Natrium-A oder Zeolith HY als wasserbindendes Mittel verwendet wird.

Die WO 2008/123928 beschreibt ein Verfahren zur Herstellung von Di-(n-butyl)-terephthalat aus Terephthalsäure und n-Butanol, wobei die Veresterung mit einem 1,25- bis 4-fachen molaren Überschuss an n-Butanol bei Normaldruck und einer Reaktionstemperatur zwischen 110 und 220 °C unter Verwendung eines Veresterungskatalysators durchgeführt wird. Konkret wird dieses Verfahren aufgrund der Siedetemperatur des n-Butanols von 117 °C bevorzugt bei einer Reaktionstemperatur von 115 bis 150 °C (d. h. im Wesentlichen unter Rückfluss) durchgeführt, wobei als Veresterungskatalysator bevorzugt eine Sulfonsäure oder Schwefelsäure eingesetzt wird. Während der Reaktion wird der Reaktionszone kontinuierlich n-Butanol zugeführt. Das während der Reaktion entstehende Wasser wird destillativ als azeotropes Gemisch abgeführt. In einigen der Ausführungsbeispiele wird Stickstoff durch die Reaktionsmischung geleitet, wobei diese Maßnahme jedoch keinen erkennbaren positiven Einfluss auf die Ausbeute, Reinheit oder Farbzahl des erhaltenen Veresterungsproduktes hat.

JP 2003160535 A beschreibt ein Verfahren zur Herstellung ungesättigter dibasischer Ester, bei dem ein Alkohol mit einer ungesättigten dibasischen Carbonsäure oder einem ungesättigten dibasischen Carbonsäureanhydrid in Anwesenheit und Abwesenheit eines organischen Lösungsmittels und in Gegenwart von para-Toluolsulfonsäure als Veresterungskatalysator bei einer Reaktionstemperatur im Bereich von 40°C bis 160°C und unter Zuführung eines Stickstoffstroms zum Reaktionssystem umgesetzt wird, wobei das Reaktionswasser als azeotrope Mischung zusammen mit dem Alkohol und/oder gegebenenfalls zusammen mit dem organischen Lösungsmittel abdestilliert wird. Nach beendeter Reaktion wird die Reaktionsmischung durch Zugabe einer wässrigen Natriumhydroxid-Lösung neutralisiert.

Der vorliegenden Erfindung liegt die Aufgabe zugrunde, ein verbessertes Verfahren zur Herstellung von Carbonsäureestern zur Verfügung zu stellen, die sich für einen Einsatz als Weichmacher eignen. Dabei soll vorzugsweise ein möglichst vollständiger Umsatz nach kurzer Reaktionszeit und damit eine hohe Raum-Zeit-Ausbeute erzielt werden. Des Weiteren soll das Verfahren kostengünstig und technisch einfach durchzuführen sein, z. B. durch die Verwendung eines preiswerten Katalysators und der Vermeidung aufwändiger Aufarbeitungsschritte, womit die oben beschriebenen Nachteile, die sich u. A. bei der Verwendung von Tetraalkyltitanaten als Veresterungskatalysatoren ergeben, weitgehend vermieden werden können. Die erhaltenen Carbonsäureester sollen sich trotzdem durch gute Produkteigenschaften, speziell für eine Anwendung als Weichmacher, auszeichnen. Dazu zählt für Anwendungen in Bereichen, bei denen die optischen Eigenschaften der weichgemachten Kunststoffe von Bedeutung sind, eine möglichst geringe Färbung der Carbonsäureester, die sich beispielsweise an einer niedrigen Farbzahl zeigt.

Überraschenderweise wurde nun gefunden, dass diese Aufgabe gelöst wird, wenn man die Veresterung zur Herstellung der als Weichmacher geeigneten Carbonsäureester bei hohen Temperaturen in Gegenwart einer organischen Sulfonsäure, speziell Methansulfonsäure mit einem Sulfatgehalt von höchstens 50 ppm, als Katalysator und in Gegenwart eines inerten Gases durchführt, wobei der zur Veresterung eingesetzte Alkohol als Schleppmittel für das gebildete Reaktionswasser dient und nach Wasserabtrennung in die Reaktion zurückgeführt wird. In einer speziellen Ausführung wird als Katalysator Methansulfonsäure mit einem Sulfatgehalt von höchstens 50 ppm und einem geringen Gesamtchlorgehalt eingesetzt.

### ZUSAMMENFASSUNG DER ERFINDUNG

Gegenstand der Erfindung ist ein Verfahren zur Herstellung von Carbonsäureestern, bei dem man in einem aus einem oder mehreren Reaktoren bestehenden Reaktionssystem ein Reaktionsgemisch umsetzt, das wenigstens eine Carbonsäure und/oder wenigstens ein Carbonsäureanhydrid und wenigstens einen Alkohol R¹-OH und/oder wenigstens einen Alkohol R²-[O-X]ₙ-OH umfasst, worin
- R¹: ausgewählt ist unter unverzweigten und verzweigten C₅-C₁₃-Alkylresten und C₅-C₆-Cycloalkylresten, wobei die Cycloalkylreste unsubstituiert sind oder durch wenigstens einen C₁-C₁₀-Alkylrest substituiert sein können,
- R²: ausgewählt ist unter unverzweigten C₁-C₁₃-Alkylresten und verzweigten C₃-C₁₃-Alkylresten,
- X: für eine unverzweigte C₂-C₅-Alkylengruppe oder verzweigte C₃-C₅-Alkylengruppe steht und
- n: die Werte 1, 2 oder 3 aufweist,
mit der Maßgabe, dass die Umsetzung
- in Gegenwart wenigstens eines Katalysators, der ausgewählt ist unter Methansulfonsäure, wobei die Methansulfonsäure einen Sulfatgehalt von höchstens 50 ppm aufweist,
- unter Zuführung eines unter den Reaktionsbedingungen inerten Gases zum Reaktionssystem,
- bei einer Temperatur des Reaktionsgemischs von 125 °C bis 240 °C und
- unter destillativer Abtrennung wenigstens eines Teils des während der Reaktion gebildeten Wassers in Form einer azeotropen Mischung mit dem eingesetzten Alkohol R¹-OH und/oder R²-[O-X]ₙ-OH,
erfolgt, wobei der abdestillierte Alkohol R¹-OH und/oder R²-[O-X]ₙ-OH zumindest teilweise in das Reaktionssystem zurückgeführt wird.

Eine bevorzugte Ausführungsform der Erfindung betrifft ein Verfahren zur Herstellung von Carbonsäureestern, bei dem man in einem aus einem oder mehreren Reaktoren bestehenden Reaktionssystem ein Reaktionsgemisch umsetzt, das wenigstens eine Carbonsäure und/oder wenigstens ein Carbonsäureanhydrid und wenigstens einen Alkohol R¹-OH umfasst, worin R¹ ausgewählt ist unter unverzweigten und verzweigten C₅-C₁₃-Alkylresten und C₅-C₆-Cycloalkylresten, wobei die Cycloalkylreste unsubstituiert sind oder durch wenigstens einen C₁-C₁₀-Alkylrest substituiert sein können, mit der Maßgabe, dass die Umsetzung
- in Gegenwart wenigstens eines Katalysators, der ausgewählt ist unter Methansulfonsäure, wobei die Methansulfonsäure einen Sulfatgehalt von höchstens 50 ppm aufweist,
- unter Zuführung eines unter den Reaktionsbedingungen inerten Gases zum Reaktionssystem,
- bei einer Temperatur des Reaktionsgemischs von 125 bis 240 °C und
- unter destillativer Abtrennung wenigstens eines Teils des während der Reaktion gebildeten Wassers in Form einer azeotropen Mischung mit dem eingesetzten Alkohol R¹-OH,
erfolgt, wobei der abdestillierte Alkohol R¹-OH zumindest teilweise in das Reaktionssystem zurückgeführt wird.

In einer speziellen Ausführungsform dient das erfindungsgemäße Verfahren zur Herstellung von Estern der Terephthalsäure, ganz speziell zur Herstellung von Bis(2-ethylhexyl)terephthalat (DOTP) durch Umsetzung von Terephthalsäure mit 2-Ethylhexanol.

In einer weiteren speziellen Ausführungsform dient das erfindungsgemäße Verfahren zur Herstellung von Estern der Essigsäure mit Alkoxyalkanolen, ganz speziell zur Herstellung von 2-Butoxyethylacetat, 2-(2-Butoxyethoxy)-ethylacetat, 1-Methoxy-2-propylacetat und 3-Methoxypropylacetat durch Umsetzung von 2-Butoxyethanol, 2-(2-Butoxy-ethoxy)-ethanol, 1-Methoxy-2-propanol oder 3-Methoxypropanol mit Essigsäure oder Essigsäureanhydrid.

Die so erhaltenen Carbonsäureester können als Weichmacher oder als Komponente in einer Weichmacherzusammensetzung für thermoplastische Polymere und Elastomere verwendet werden.

### BESCHREIBUNG DER ERFINDUNG

Das erfindungsgemäße Verfahren hat folgende Vorteile:
- Es wird die Herstellung von als Weichmacher geeigneten Carbonsäureestern bei kurzen Reaktionszeiten, d. h. mit einer hohen Raum-Zeit-Ausbeute ermöglicht.
- Die Carbonsäureester werden trotz der relativ drastischen Reaktionsbedingungen in hohen Ausbeuten und guten Selektivitäten erhalten.
- Obwohl eine Brönstedsäure als Katalysator eingesetzt wird, wird die Bildung unerwünschter Nebenprodukte, speziell von Ethern des zur Veresterung eingesetzten Alkohols und von Olefinen aus der Wassereliminierung des Alkohols, nur in sehr geringem Umfang beobachtet.
- Auf den Einsatz aufwändiger Maßnahmen zur Reinigung der nach dem erfindungsgemäßen Verfahren erhaltenen Carbonsäureester kann in der Regel verzichtet werden. Dies gilt speziell für den Einsatz von Adsorbentien zur Erzielung weniger gefärbter Produkte.
- Auf den Einsatz externer organischer Lösungsmittel, d. h. von als Lösungsmittel wirksamen Komponenten, die von den zur Herstellung der Carbonsäureester eingesetzten Edukten und den gebildeten Reaktionsprodukten verschieden sind, kann in der Regel verzichtet werden.
- Das erfindungsgemäße Verfahren eignet sich speziell zur Herstellung von Estern der Terephthalsäure, Trimellitsäure, Benzoesäure und Estern alicyclischer und aliphatischer Carbonsäuren die aufgrund ihrer günstigen toxikologischen Eigenschaften von großer Bedeutung für den Einsatz als Weichmacher sind.
- Die erhaltenen Carbonsäureester sind nicht oder nur gering gefärbt und zeichnen sich durch eine niedrige Hazen-Farbzahl (bestimmbar nach DIN/EN/ISO 6271-2) aus. Diese ist in der Regel wenigstens gleich gut oder besser wie bei Produkten, die nach dem wesentlich aufwändigeren Verfahren mittels Katalyse durch Tetraalkyltitanate erhalten werden.

Im Rahmen der vorliegenden Erfindung umfasst der Ausdruck "C₁-C₁₀-Alkyl" unverzweigte Alkylgruppen mit 1 bis 10 Kohlenstoffatomen sowie verzweigte Alkylgruppen mit 3 bis 10 Kohlenstoffatomen. Dazu zählen Methyl, Ethyl, Propyl, Isopropyl, n-Butyl, Isobutyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, n-Octyl, Isooctyl, 2-Ethylhexyl, n-Nonyl, Isononyl, 2-Propylhexyl, n-Decyl, Isodecyl, 2-Propylheptyl und dergleichen. Vorzugsweise handelt es sich dabei um unverzweigte C₁-C₈-Alkylgruppen oder verzweigte C₃-C₈-Alkylgruppen. Besonders bevorzugt handelt es sich dabei um unverzweigte C₁-C₅-Alkylgruppen oder verzweigte C₃-C₅-Alkylgruppen.

Der Ausdruck "C₅-C₁₃-Alkyl" umfasst unverzweigte und verzweigte C₅-C₁₃-Alkylgruppen. Vorzugsweise ist C₅-C₁₃-Alkyl ausgewählt unter n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 1-Ethylpentyl, 2-Ethylpentyl, 1-Propylbutyl, 1-Ethyl-2-methylpropyl, n-Octyl, Isooctyl, 2-Ethylhexyl, n-Nonyl, Isononyl, 2-Propylhexyl, n-Decyl, Isodecyl, 2-Propylheptyl, n-Undecyl, Isoundecyl, n-Dodecyl, Isododecyl, n-Tridecyl oder Isotridecyl und dergleichen. Besonders bevorzugt steht C₅-C₁₃-Alkyl für n-Octyl, n-Nonyl, Isononyl, 2-Ethylhexyl, Isodecyl, 2-Propylheptyl, n-Undecyl, Isoundecyl, n-Tridecyl oder Isotridecyl.

Der Ausdruck "C₁-C₁₃-Alkyl" umfasst unverzweigte Alkylgruppen mit 1 bis 13 Kohlenstoffatomen sowie verzweigte Alkylgruppen mit 3 bis 13 Kohlenstoffatomen. Vorzugsweise ist C₁-C₁₃-Alkyl ausgewählt unter Methyl, Ethyl, Propyl, Isopropyl,
n-Butyl, Isobutyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 1-Ethylpentyl, 2-Ethylpentyl, 1-Propylbutyl, 1-Ethyl-2-methylpropyl, n-Octyl, Isooctyl, 2-Ethylhexyl, n-Nonyl, Isononyl, 2-Propylhexyl, n-Decyl, Isodecyl, 2-Propylheptyl, n-Undecyl, Isoundecyl, n-Dodecyl, Isododecyl, n-Tridecyl oder Isotridecyl und dergleichen. Besonders bevorzugt steht C₁-C₁₃-Alkyl für unverzweigte C₁-C₉-Alkylgruppen oder verzweigte C₃-C₉-Alkylgruppen, insbesondere für Methyl, Ethyl, Propyl, n-Butyl, n-Pentyl, n-Hexyl, n-Heptyl, n-Octyl, 2-Ethylhexyl, n-Nonyl oder Isononyl.

Der Ausdruck "C₁-C₁₃-Alkyl" beinhaltet in seiner Definition auch die Ausdrücke "C₃-C₁₃-Alkyl", "C₁-C₉-Alkyl" und "C₃-C₉-Alkyl".

Der Ausdruck "C₅-C₆-Cycloalkyl" umfasst im Sinne der vorliegenden Erfindung cyclische Kohlenwasserstoffe mit 5 bis 6, insbesondere mit 6 Kohlenstoffatomen. Dazu zählen Cyclopentyl oder Cyclohexyl.

Substituierte C₅-C₆-Cycloalkylgruppen können, in Abhängigkeit von ihrer Ringgröße, einen oder mehrere (z. B. 1, 2, 3, 4 oder 5) C₁-C₁₀-Alkylsubstituenten aufweisen. Beispiele für C₅-C₆-Cycloalkylgruppen sind 2- und 3-Methylcyclopentyl, 2- und 3-Ethylcyclopentyl, 2-, 3- und 4-Methylcyclohexyl, 2-, 3- und 4-Ethylcyclohexyl, 2-, 3- und 4-Propylcyclohexyl, 2-, 3- und 4-Isopropylcyclohexyl, 2-, 3- und 4-Butylcyclohexyl, 2-, 3- und 4-sec.-Butylcyclohexyl, 2-, 3- und 4-tert.-Butylcyclohexyl und dergleichen.

Im Rahmen der vorliegenden Erfindung umfasst der Ausdruck "C₂-C₅-Alkylen" unverzweigte zweiwertige Kohlenwasserstoffreste mit 2 bis 5 Kohlenstoffatomen sowie verzweigte zweiwertige Kohlenwasserstoffreste mit 3 bis 5 Kohlenstoffatomen. Dazu zählen beispielsweise 1,2-Ethylen, 1,3-Propylen, 1,2-propylen, 1-Methyl-1,2-ethylen, 1,4-Butylen, 1-Methyl-1,3-propylen, 2-Methyl-1,3-propylen, 1,5-Pentylen, 1-Methyl-1,4-butylen, 2-Methyl-1,4-butylen und dergleichen. Bevorzugt handelt es sich bei "C₂-C₅-Alkylen" um unverzweigte C₂-C₄-Alkylengruppen oder verzweigte C₃-C₄-Alkylengruppen, besonders bevorzugt um 1,2-Ethylen und 1,3-Propylen.

Der Ausdruck "C₂-C₅-Alkylen" beinhaltet in seiner Definition auch die Ausdrücke "C₃-C₅-Alkylen", "C₂-C₄-Alkylen", "C₃-C₄-Alkylen" und "C₂-C₃-Alkylen".

Unter "Reaktionssystem" wird im Sinne der Erfindung ein Reaktor oder eine Anordnung von mehreren Reaktoren verstanden. Mehrere Reaktoren sind vorzugsweise hintereinander geschaltet. Das erfindungsgemäße Verfahren kann diskontinuierlich oder kontinuierlich durchgeführt werden, wird aber vorzugsweise kontinuierlich durchgeführt.

Bei den Reaktoren kann es sich um beliebige Reaktoren handeln, die zur Durchführung von chemischen Umsetzungen in flüssiger Phase geeignet sind.

Als Reaktoren sind nicht rückvermischte Reaktoren, wie Rohrreaktoren oder mit Einbauten versehene Verweilzeitbehälter, vorzugsweise aber rückvermischte Reaktoren, wie Rührkessel, Schlaufenreaktoren, Strahlschlaufenreaktoren oder Strahldüsenreaktoren geeignet. Es können aber auch Kombinationen aus aufeinander folgenden rückvermischten Reaktoren und nicht rückvermischten Reaktoren verwendet werden.

Gegebenenfalls können auch mehrere Reaktoren in einer mehrstufigen Apparatur zusammengefasst werden. Solche Reaktoren sind zum Beispiel Schlaufenreaktoren mit eingebauten Siebböden, kaskadierte Behälter, Rohrreaktoren mit Zwischeneinspeisung oder Rührkolonnen.

Vorzugsweise werden Rührkesselreaktoren verwendet. Die Rührkesselreaktoren bestehen meist aus metallischen Werkstoffen, wobei Edelstahl bevorzugt ist. Der Reaktionsansatz wird vorzugsweise mit Hilfe eines Rührers oder einer Umlaufpumpe intensiv vermischt.

In einer bevorzugten Ausführungsform wird das erfindungsgemäße Verfahren in einem einzelnen Rührkessel durchgeführt. In einer weiteren bevorzugten Ausführungsform wird das erfindungsgemäße Verfahren in wenigstens zwei Rührkesseln durchgeführt, die miteinander in Form einer Kaskade verbunden sind. Speziell bei kontinuierlicher Verfahrensführung kann es für eine möglichst vollständige Umsetzung zweckmäßig sein, mehrere Reaktoren in Form einer Kaskade zu verbinden. Die einzelnen Reaktoren werden vom Reaktionsgemisch nacheinander durchlaufen, wobei der Ablauf des ersten Reaktors dem zweiten Reaktor, der Ablauf des zweiten Reaktors dem dritten Reaktor usw. zugeführt wird. Die Kaskade kann z. B. 2 bis 10 Reaktoren umfassen, wobei 2, 3, 4 oder 5 Reaktoren bevorzugt sind.

Bei diskontinuierlicher Verfahrensdurchführung können Carbonsäure und/oder Carbonsäureanhydrid und Alkohol R¹-OH und/oder R²-[O-X]ₙ-OH und der Katalysator gleichzeitig oder nacheinander in den Reaktor eingefüllt werden. Der Katalysator kann in reiner Form oder als Lösung, bevorzugt gelöst in einem der Einsatzstoffe, zu Beginn oder erst nach Erreichen der Reaktionstemperatur eingebracht werden. Carbonsäureanhydride reagieren häufig mit Alkoholen bereits autokatalytisch, d. h. unkatalysiert zu den entsprechenden Ester-carbonsäuren (Halbestern), beispielsweise Phthalsäureanhydrid zum Phthalsäuremonoester. Daher ist ein Katalysator häufig erst nach dem ersten Reaktionsschritt erforderlich.

Bei kontinuierlicher Verfahrensdurchführung führt man Ströme der Edukte und des Katalysators in den Reaktor bzw. bei Verwendung einer Reaktorkaskade vorzugsweise in den ersten Reaktor der Kaskade ein. Die Verweilzeit im Reaktor bzw. den einzelnen Reaktoren wird dabei durch das Volumen der Reaktoren und den Mengenstrom der Edukte bestimmt.

Das erfindungsgemäße Verfahren erfolgt unter Zuführung eines unter den Reaktionsbedingungen inerten Gases zum Reaktionssystem. Dazu kann das inerte Gas in den Gasraum des Reaktionssystems oder in das flüssige Reaktionsgemisch geleitet werden. Bevorzugt erfolgt die Zuführung des inerten Gases zum Reaktionssystem so, dass eine große Austauschfläche zwischen dem flüssigen Reaktionsgemisch und dem inerten Gas geschaffen wird. Die Behandlung mit dem inerten Gas während der Umsetzung hat einen Stripp-Effekt und vervollständigt die Entfernung des Reaktionswassers. Weiterhin ist es möglich, durch Zuführung eines erhitzten inerten Gases Energie in das Reaktionssystem einzutragen. In dieser Ausführung kann der Energieeintrag über den Reaktormantel entsprechend gedrosselt werden. Dadurch kann vorteilhafterweise eine Überhitzung des Reaktionsgemisches in der Nähe des Reaktormantels und die Bildung von Nebenprodukten verringert werden.

In bevorzugten Ausführungsformen wird das inerte Gas unter der Flüssigkeitsoberfläche in das siedende Reaktionsgemisch eingeführt, so dass es das Reaktionsgemisch durchperlt. Der Druck des inerten Gases muss ausreichend hoch sein, um den hydrostatischen Druck des Reaktionsgemisches oberhalb der Inertgas-Einspeisung zu überwinden. Z. B. kann man das inerte Gas 20 bis 50 cm unterhalb der Flüssigkeitsoberfläche des Reaktionsgemisches einführen.

Das inerte Gas kann über beliebige geeignete Vorrichtungen eingespeist werden. Dazu zählen z. B. Begasungslanzen oder Düsen. Die Düsen können am oder in der Nähe des Reaktorbodens vorgesehen sein. Die Düsen können hierzu als Öffnungen einer den Reaktor umgebenden Hohlkammer ausgebildet sein. Alternativ können Tauchdüsen mit geeigneten Zuleitungen eingesetzt werden. Mehrere Düsen können z. B. in Form eines Kranzes angeordnet sein. Die Düsen können nach oben oder nach unten weisen. Die Düsen weisen vorzugsweise schräg nach unten.

Vorzugsweise wird das Reaktionsgemisch durchmischt, um einen Austausch von Reaktionsgemisch im Reaktorbereich unterhalb der Einspeisung des inerten Gases mit Reaktionsgemisch im Reaktorbereich oberhalb der Einspeisung des inerten Gases zu bewirken. Zur Vermischung eignen sich beispielsweise Rührer oder Umlaufpumpe. In einer speziellen Variante wird ein so genannter Begasungsrührer zur Zuführung des inerten Gases und zur Vermischung des Reaktionsgemischs eingesetzt.

Wird das erfindungsgemäße Verfahren in wenigstens zwei Rührkesseln durchgeführt, die miteinander in Form einer Kaskade verbunden sind, so werden vorzugsweise alle Reaktoren der Kaskade von dem inerten Gas durchströmt. Wenn mehr als ein Reaktor mit dem inerten Gas behandelt wird, kann dieses parallel zu den einzelnen Reaktoren geführt werden oder das inerte Gas passiert mehrere Reaktoren nacheinander. Es sind auch Kombinationen denkbar, in denen zwei oder mehrere Reaktoren mit frischem inerten Gas durchperlt werden und der das inerte Gas enthaltende Dampf aus wenigstens einem der Reaktoren durch wenigstens einen weiteren Reaktor geleitet wird.

Beispielsweise kann man in einer Kaskade von n Reaktoren frisches inertes Gas in den in Stromrichtung letzten Reaktor einführen, den das inerte Gas enthaltenden Brüden aus dem n-ten Reaktor sammeln und dampfförmig in das Reaktionsgemisch im (n-ersten) Reaktor einführen, usw.

Die Veresterung erfolgt erfindungsgemäß in Gegenwart eines inerten Gases. Unter einem inerten Gas wird ein Gas verstanden, welches unter den gegebenen Verfahrensbedingungen keine Reaktionen mit den an der Reaktion beteiligten Edukten, Reagenzien, Lösungsmitteln oder den entstehenden Produkten eingeht. Geeignete inerte Gase sind z. B. Stickstoff, Helium, Argon, etc. Bevorzugt wird als inertes Gas Stickstoff eingesetzt.

Erfindungsgemäß erfolgt das Verfahren unter destillativer Abtrennung wenigstens eines Teils des während der Reaktion gebildeten Wassers in Form einer azeotropen Mischung mit dem eingesetzten Alkohol R¹-OH und/oder R²-[O-X]ₙ-OH, der anschließend zumindest teilweise in das Reaktionssystem zurückgeführt wird. Dazu entnimmt man aus dem Reaktionssystem einen Brüden, kondensiert diesen, trennt das Kondensat in eine wässrige Phase und eine Alkoholphase und führt die Alkoholphase zumindest teilweise in das Reaktionssystem zurück. "Rückführung in das Reaktionssystem" bedeutet, dass die Alkoholphase in wenigstens einen beliebigen Reaktor des Reaktionssystems geleitet wird.

Zur Kondensation bzw. partiellen Kondensation des Brüdens können alle geeigneten Kondensatoren verwendet werden. Diese können mit beliebigen Kühlmedien gekühlt werden. Kondensatoren mit Luftkühlung und/oder Wasserkühlung sind bevorzugt, wobei die Luftkühlung besonders bevorzugt ist.

Das erhaltene Kondensat wird einer Phasentrennung in eine wässrige Phase und eine organische Phase unterzogen. Üblicherweise wird das Kondensat hierzu in einen Phasenscheider (Dekanter) geleitet, wo es durch mechanisches Absetzen in zwei Phasen zerfällt, die getrennt abgezogen werden können. Die wässrige Phase wird abgetrennt und kann, gegebenenfalls nach Aufarbeitung, verworfen oder als Strippwasser bei der Nachbehandlung des Esters verwendet werden.

Der Brüden aus den einzelnen Reaktoren einer Kaskade kann vereinigt werden und gemeinsam kondensiert werden. Gegebenenfalls kann man jeweils mehrere Reaktoren der Kaskade zu einer Untereinheit zusammenfassen, wobei dann jeweils die Untereinheiten mit einem Kondensator gekoppelt sind. Es besteht daneben weiterhin die Möglichkeit, jeden Reaktor der Kaskade mit einem Kondensator zu koppeln.

Die zurückzuführende Alkoholphase kann in einen beliebigen Reaktor einer Kaskade geleitet oder auf mehrere Reaktoren der Kaskade aufgeteilt werden. Es ist jedoch bevorzugt, die zurückzuführende Alkoholphase nicht in den letzten Reaktor der Kaskade zu leiten. Vorzugsweise leitet man die zurückzuführende Alkoholphase ausschließlich oder überwiegend in den ersten Reaktor der Kaskade.

Für die Rückführung der Alkoholphase in das Reaktionssystem gibt es verschiedene Möglichkeiten. Eine Möglichkeit ist, die organische Phase, gegebenenfalls nach Erwärmen, in das flüssige Reaktionsgemisch zu pumpen.

Zur thermischen Optimierung des Verfahrens kann man die Alkoholphase über eine Kolonne (so genannte Rückalkohol-Kolonne) in das Reaktionssystem zurückführen. In dieser Rückalkohol-Kolonne wird die rückgeführten Alkoholphase zumindest einem Teil des Brüdens entgegengeführt. Zweckmäßigerweise führt man die Alkoholphase am Kopf oder im oberen Bereich in die Rückalkohol-Kolonne ein. Das ablaufende Kondensat der Rückalkohol-Kolonne gelangt in das Reaktionssystem zurück. Bei Verwendung einer Reaktorkaskade wird das ablaufende Kondensat der Rückalkohol-Kolonne vorzugsweise in den ersten Reaktor eingeführt. Die Rückführung der Alkoholphase über die Rückalkohol-Kolonne weist den Vorteil auf, dass die rückgeführte Alkoholphase vorerwärmt und von Wasserspuren befreit wird, die nach der Phasentrennung in der organischen Phase verblieben sind bzw. gemäß ihrer thermodynamischen Löslichkeit in der organischen Phase gelöst sind. Bei der Rückalkohol-Kolonne kann es sich beispielsweise um eine Bodenkolonne, Packungskolonne oder Füllkörperkolonne handeln. Eine geringe Trennstufenzahl ist im Allgemeinen ausreichend. Geeignet ist z. B. eine Kolonne mit 2 bis 10 theoretischen Trennstufen. Bei Verwendung einer Reaktorkaskade verlässt der Brüden vorzugsweise zumindest den ersten Reaktor über die Rückalkohol-Kolonne. Ein oder mehrere oder alle weiteren Reaktoren können ebenfalls einen Brüdenabzug zur Rückalkohol-Kolonne aufweisen.

Der wenigstens eine Alkohol R¹-OH und/oder der wenigstens eine Alkohol R²-[O-X]ₙ-OH wird vorzugsweise im stöchiometrischen Überschuss gegenüber den Carboxylgruppen eingesetzt. Dabei wird angenommen, dass ein Carbonsäureanhydrid zwei zu veresternde Carboxylgruppen aufweist. Der wenigstens eine Alkohol R¹-OH und/oder R²-[O-X]ₙ-OH wird besonders bevorzugt in einem 1 bis 100%igen molaren Überschuss, insbesondere in einem 5 bis 50%igen molaren Überschuss, speziell in einem 7 bis 15%igen molaren Überschuss eingesetzt.

Der Katalystor wird vorzugsweise in einer Menge von 0,5 bis 5 Mol%, besonders bevorzugt von 1 bis 2 Mol%, bezogen auf die Molmenge der Carboxylgruppen eingesetzt.

Geeignete Veresterungskatalysatoren sind Methansulfonsäure und Toluolsulfonsäure. Erfindungsgemäß wird als Veresterungskatalysator Methansulfonsäure eingesetzt, wobei die Methansulfonsäure einen Sulfatgehalt von höchstens 50 ppm aufweist. Der Katalysator kann als Reinsubstanz oder in Form einer wässrigen Lösung eingesetzt werden.

Im Rahmen der vorliegenden Erfindung bezeichnet der Ausdruck "Gesamtchlorgehalt" die Summe aus dem Gehalt an freiem sowie organisch oder anorganisch gebundenem Chlor.

Bevorzugt weist die eingesetzte Methansulfonsäure einen Gesamtchlorgehalt von höchstens 20 ppm, bevorzugt von höchstens 5 ppm, insbesondere von höchstens 1 ppm, auf.

Bevorzugt weist die eingesetzte Methansulfonsäure einen Sulfatgehalt von höchstens 20 ppm, auf.

Eine besonders geeignete reine Methansulfonsäure ist erhältlich nach dem Verfahren wie in der WO 0050351 beschrieben. Eine solche reine MSA ist kommerziell unter der Bezeichnung Lutropur ® der BASF SE erhältlich, entweder als 70%ige wässrige Lösung (Lutropur® MSA), oder als wasserfreie MSA (Lutropur® MSA100).

Die Veresterung wird bevorzugt in einem Temperaturbereich von 130 bis 235 °C, insbesondere von 135 bis 230 °C, durchgeführt. Nach dem erfindungsgemäßen Verfahren kann die Veresterung auch bei noch höheren Temperaturen, speziell von wenigstens 150 °C, spezieller von wenigstens 170 °C durchgeführt werden.

Die optimalen Temperaturen hängen von den Einsatzstoffen, dem Reaktionsfortschritt und der Katalysatorkonzentration ab. Sie können für jeden Einzelfall durch Versuche leicht ermittelt werden. Es ist zur Entfernung des Reaktionswassers erforderlich, dass der Alkohol aus dem Reaktionsgemisch abdestillieren kann. Die gewünschte Temperatur oder der gewünschte Temperaturbereich kann durch den Druck im Reaktor eingestellt werden. Bei niedrig siedenden Alkoholen kann daher die Umsetzung bei Überdruck oder Umgebungsdruck und bei höher siedenden Alkoholen bei vermindertem Druck durchgeführt werden.

Wird zur Veresterung eine Kaskade aus mehreren Reaktoren eingesetzt, so können alle Reaktoren einer Kaskade bei gleicher Temperatur betrieben werden. Im Allgemeinen ist es aber bevorzugt, die Temperatur vom ersten zum letzten Reaktor einer Kaskade stetig zu erhöhen, wobei ein Reaktor bei gleicher oder höherer Temperatur betrieben wird, als der in Fließrichtung des Reaktionsgemisches stromaufwärts gelegene Reaktor. Zweckmäßigerweise können alle Reaktoren bei im Wesentlichen gleichem Druck betrieben werden.

Die Veresterung erfolgt vorzugsweise bei Umgebungsdruck oder unter vermindertem Druck. Bevorzugt wird die Veresterung bei einem Druck von 0,001 bis 2,0 bar, besonders bevorzugt 0,01 bis 1,1 bar durchgeführt.

Die Veresterung kann in Abwesenheit eines externen Lösungsmittels oder in Gegenwart eines organischen Lösungsmittels durchgeführt werden. Bevorzugt wird die Veresterung in Abwesenheit eines externen Lösungsmittels durchgeführt.

Falls die Veresterung in Gegenwart eines externen Lösungsmittels durchgeführt wird, handelt es sich dabei vorzugsweise um ein unter den Reaktionsbedingungen inertes organisches Lösungsmittel. Dazu gehören beispielsweise aliphatische Kohlenwasserstoffe, halogenierte aliphatische Kohlenwasserstoffe, aromatische und substituierte aromatische Kohlenwasserstoffe oder Ether. Bevorzugt ist das Lösungsmittel ausgewählt unter Pentan, Hexan, Heptan, Ligroin, Petrolether, Cyclohexan, Dichlormethan, Trichlormethan, Tetrachlormethan, Benzol, Toluol, Xylol, Chlorbenzol, Dichlorbenzolen, Dibutylether, THF, Dioxan und Mischungen davon.

Nach Beendigung der Reaktion enthält das Reaktionsgemisch, das im Wesentlichen aus dem gewünschten Ester und überschüssigem Alkohol besteht, neben dem Katalysator und/oder dessen Folgeprodukte geringe Mengen an Estercarbonsäure(n) und/oder nicht umgesetzter Carbonsäure.

Zur Aufarbeitung dieser Esterrohgemische wird der überschüssige Alkohol entfernt, die sauren Verbindungen neutralisiert und die dabei entstandenen festen Nebenprodukte abgetrennt. Die Reihenfolge der Prozessschritte kann dabei variiert werden. Dabei wird der größte Teil des nicht umgesetzten Alkohols bei Normaldruck oder im Vakuum abdestilliert. Die letzten Spuren des Alkohols können z. B. durch Wasserdampfdestillation, insbesondere im Temperaturbereich von 120 bis 225 °C unter Vakuum, entfernt werden. Die Abtrennung des Alkohols kann als erster oder als letzter Aufarbeitungsschritt erfolgen.

Die Neutralisation der sauren Stoffe, wie Carbonsäuren, Estercarbonsäuren oder gegebenenfalls der sauren Katalysatoren, erfolgt durch Zugabe von Basen, z. B. von Alkali- und/oder Erdalkalimetallcarbonaten, -hydrogencarbonaten oder -hydroxiden. Das Neutralisationsmittel kann in fester Form oder bevorzugt als Lösung, insbesondere als wässrige Lösung eingesetzt werden. Hier wird häufig Natronlauge einer Konzentration von 1 bis 30 Gew.-%, bevorzugt von 20 bis 30 Gew.-% verwendet. Das Neutralisationsmittel wird in einer Menge zugesetzt, die dem Einfachen bis dem Vierfachen, insbesondere dem Einfachen bis Zweifachen der stöchiometrisch notwendigen Menge, die durch Titration bestimmt wird, entspricht.

Bei der Destillation wird auch, soweit vorhanden, der Ether des eingesetzten Alkohols R¹-OH und/oder des eingesetzten Alkohols R²-[O-X]ₙ-OH entfernt. Dieser ist in den nach dem erfindungsgemäßen Verfahren erhaltenen Reaktionsgemischen im Allgemeinen in einer Menge von < 2 Gew.-%, bevorzugt < 1 Gew.-%, enthalten (bestimmt durch GC-Messungen an derivatisierten Proben). Der erhaltene Ether kann gewünschtenfalls durch saure Etherspaltung wieder in den Alkohol R¹-OH bzw. in den Alkohol R²-[O-X]ₙ-OH überführt werden.

Der überschüssige Alkohol R¹-OH und/oder R²-[O-X]ₙ-OH kann entweder direkt wiederverwendet werden oder weiter gereinigt werden, z. B. mittels Destillation.

Der erhaltene Carbonsäureester ist im Wesentlichen frei von festen Verunreinigungen. Er kann jedoch einer Filtration unterzogen werden, um eventuell im Reaktor befindliche Schwebstoffe zu entfernen.

Bevorzugte Alkohole R¹-OH sind C₅-C₁₃-Alkanole. Die C₅-C₁₃-Alkanole können geradkettig oder verzweigt sein oder aus Gemischen aus geradkettigen und verzweigten C₅-C₁₃-Alkanole bestehen. Zu den bevorzugten C₅-C₁₃-Alkanolen zählen beispielsweise n-Pentanol, 2-Methylbutanol, n-Hexanol, n-Heptanol, Isoheptanol, n-Octanol, Isooctanol, 2-Ethylhexanol, n-Nonanol, Isononanol, Isodecanol, 2-Propylheptanol, n-Undecanol, Isoundecanol, n-Dodecanol, Isododecanol, n-Tridecanol oder Isotridecanol sowie Gemische davon. Besonders bevorzugt sind C₇-C₁₂-Alkanole.

Als Alkohole R¹-OH besonders bevorzugte C₇-C₁₂-Alkanole können geradkettig oder verzweigt sein oder aus Gemischen aus geradkettigen und verzweigten C₇-C₁₂-Alkanolen bestehen. Zu den besonders bevorzugten C₇-C₁₂-Alkanole zählen beispielsweise n-Octanol, 2-Ethylhexanol, n-Nonanol, Isononanol, Isodecanol, 2-Propylheptanol, n-Undecanol, Isoundecanol oder n-Dodecanol sowie Gemische davon. Insbesondere wird in dem erfindungsgemäßen Verfahren als Alkohol 2-Ethylhexanol eingesetzt.

Bevorzugt werden als Alkohole R¹-OH weiterhin C₅-C₆-Cycloalkanole sowie C₅-C₁₃-Alkanole verwendet. Die C₅-C₆-Cycloalkanole sind ausgewählt unter Cyclopentanol oder Cyclohexanol sowie Gemischen davon. Bevorzugt ist Cyclohexanol. Substituierte C₅-C₆-Cycloalkanole können, in Abhängigkeit von ihrer Ringgröße, einen oder mehrere (z. B. 1, 2, 3, 4 oder 5) C₁-C₁₀-Alkylsubstituenten aufweisen. Beispiele für C₅- bis C₆-Cycloalkanole sind 2- und 3-Methylcyclopentanol, 2- und 3-Ethylcyclopentanol, 2-, 3- und 4-Methylcyclohexanol, 2-, 3- und 4-Ethylcyclohexanol, 2-, 3- und 4-Propylcyclohexanol, 2-, 3- und 4-Isopropylcyclohexanol, 2-, 3- und 4-Butylcyclohexanol, 2-, 3- und 4-sec.-Butylcyclohexanol und 2-, 3- und 4-tert.-Butylcyclohexanol.

Besonders bevorzugte C₇-C₁₂-Alkanole werden im Folgenden genauer definiert.

### Heptanol

Die im erfindungsgemäßen Verfahren eingesetzten Heptanole können geradkettig oder verzweigt sein oder aus Gemischen aus geradkettigen und verzweigten Heptanolen bestehen. Bevorzugt werden Gemische aus verzweigten Heptanolen, auch als Isoheptanol bezeichnet, verwendet, die durch die Rhodium- oder vorzugsweise Kobalt-katalysierte Hydroformylierung von Dimerpropen, erhältlich z. B. nach dem Dimersol®-Verfahren, und anschließende Hydrierung der erhaltenen Isoheptanale zu einem Isoheptanol-Gemisch, hergestellt werden. Entsprechend seiner Herstellung besteht das so gewonnene Isoheptanol-Gemisch aus mehreren Isomeren. Im Wesentlichen geradkettige Heptanole können durch die Rhodium- oder vorzugsweise Kobalt-katalysierte Hydroformylierung von 1-Hexen und anschließende Hydrierung des erhaltenen n-Heptanals zu n-Heptanol erhalten werden. Die Hydroformylierung von 1-Hexen bzw. Dimerpropen kann nach an sich bekannten Verfahren erfolgen: Bei der Hydroformylierung mit homogen im Reaktionsmedium gelösten Rhodiumkatalysatoren können sowohl unkomplexierte Rhodiumcarbonyle, die in situ unter den Bedingungen der Hydroformylierungsreaktion im Hydroformylierungsreaktionsgemisch unter Einwirkung von Synthesegas z. B. aus Rhodiumsalzen gebildet werden, als auch komplexe Rhodiumcarbonylverbindungen, insbesondere Komplexe mit organischen Phosphinen, wie Triphenylphosphin, oder Organophosphiten, vorzugsweise chelatisierenden Biphosphiten, wie z. B. in US-A 5288918 beschrieben, als Katalysator verwendet werden. Bei der Kobalt-katalysierten Hydroformylierung dieser Olefine werden im Allgemeinen homogen im Reaktionsgemisch lösliche Kobaltcarbonyl-Verbindungen eingesetzt, die sich unter den Bedingungen der Hydroformylierungsreaktion unter Einwirkung von Synthesegas in situ aus Kobaltsalzen bilden. Wird die Kobalt-katalysierte Hydroformylierung in Gegenwart von Trialkyl- oder Triarylphosphinen ausgeführt, bilden sich als Hydroformylierungsprodukt direkt die gewünschten Heptanole, so dass keine weitere Hydrierung der Aldehydfunktion mehr benötigt wird.

Zur Kobalt-katalysierten Hydroformylierung des 1-Hexens bzw. der Hexenisomerengemische eignen sich beispielsweise die in Falbe, New Syntheses with Carbon Monoxide, Springer, Berlin, 1980 auf den Seiten 162 - 168, erläuterten industriell etablierten Verfahren, wie das Ruhrchemie-Verfahren, das BASF-Verfahren, das Kuhlmann-Verfahren oder das Shell-Verfahren. Während das Ruhrchemie-, BASF- und das Kuhlmann-Verfahren mit nicht ligandmodifizierten Kobaltcarbonyl-Verbindungen als Katalysatoren arbeiten und dabei Hexanal-Gemische erhalten, verwendet das Shell-Verfahren (DE-A 1593368) Phosphin- oder Phosphit-Ligand-modifizierte Kobaltcarbonyl-Verbindungen als Katalysator, die aufgrund ihrer zusätzlichen hohen Hydrieraktivität direkt zu den Hexanolgemischen führen. Vorteilhafte Ausgestaltungen zur Durchführung der Hydroformylierung mit nicht-ligandmodifizierten Kobaltcarbonylkomplexen werden in DE-A 2139630, DE-A 2244373, DE-A 2404855 und WO 01014297 detailliert beschrieben.

Zur Rhodium-katalysierten Hydroformylierung des 1-Hexens bzw. der Hexenisomerengemische kann das industriell etablierte Rhodium-Niederdruck-Hydroformylierungs-verfahren mit Triphenylphosphinligand-modifizierten Rhodiumcarbonylverbindungen angewandt werden, wie es Gegenstand von US-A 4148830 ist. Vorteilhaft können zur Rhodium-katalysierten Hydroformylierung langkettiger Olefine, wie der nach den vorstehend genannten Verfahren erhaltenen Hexenisomerengemische nicht-ligand-modifizierte Rhodiumcarbonylverbindungen als Katalysator dienen, wobei im Unterschied zum Niederdruckverfahren ein höherer Druck von 80 bis 400 bar einzustellen ist. Die Durchführung solcher Rhodium-Hochdruck-Hydroformylierungs-verfahren wird in z. B. EP-A 695734, EP-B 880494 und EP-B 1047655 beschrieben.

Die nach Hydroformylierung der Hexen-Isomerengemische erhaltenen Isoheptanalgemische werden auf an sich herkömmliche Weise zu Isoheptanolgemischen katalytisch hydriert. Bevorzugt werden hierzu heterogene Katalysatoren verwendet, die als katalytisch aktive Komponente Metalle und/oder Metalloxide der VI. bis VIII. sowie der I. Nebengruppe des Periodensystems der Elemente, insbesondere Chrom, Molybdän, Mangan, Rhenium, Eisen, Kobalt, Nickel und/oder Kupfer, gegebenenfalls abgeschieden auf einem Trägermaterial wie Al₂O₃, SiO₂ und/oder TiO₂, enthalten. Solche Katalysatoren werden z. B. in DE-A 3228881, DE-A 2628987 und DE-A 2445303 beschrieben. Besonders vorteilhaft wird die Hydrierung der Isoheptanale mit einem Überschuss an Wasserstoff von 1,5 bis 20 % über der stöchiometrisch zur Hydrierung der Isoheptanale benötigten Wasserstoffmenge, bei Temperaturen von 50 bis 200 °C und bei einem Wasserstoffdruck von 25 bis 350 bar durchgeführt und zur Vermeidung von Nebenreaktionen dem Hydrierzulauf gemäß DE-A 2628987 eine geringe Menge Wasser, vorteilhaft in Form einer wässrigen Lösung eines Alkalimetallhydroxids oder -carbonats entsprechend der Lehre von WO 01087809 zugefügt.

### Octanol

2-Ethylhexanol, das für lange Jahre der in den größten Mengen produzierte Weichmacheralkohol war, kann über die Aldolkondensation von n-Butyraldehyd zu 2-Ethylhexenal und dessen anschließende Hydrierung zu 2-Ethylhexanol gewonnen werden (s. Ullmann's Encyclopedia of Industrial Chemistry; 5. Auflage, Bd. A 10, S. 137 - 140, VCH Verlagsgesellschaft GmbH, Weinheim 1987).

Im Wesentlichen geradkettige Octanole können durch die Rhodium- oder vorzugsweise Kobalt-katalysierte Hydroformylierung von 1-Hepten und anschließende Hydrierung des erhaltenen n-Octanals zu n-Octanol erhalten werden. Das dazu benötigte 1-Hepten kann aus der Fischer-Tropsch-Synthese von Kohlenwasserstoffen gewonnen werden.

Bei dem Alkohol Isooctanol handelt es sich im Unterschied zu 2-Ethylhexanol oder n-Octanol, bedingt durch seine Herstellungsweise, nicht um eine einheitliche chemische Verbindung sondern um ein Isomerengemisch aus unterschiedlich verzweigten C₈-Alkoholen, beispielsweise aus 2,3-Dimethyl-1-hexanol, 3,5-Dimethyl-1-hexanol, 4,5-Dimethyl-1-hexanol, 3-Methyl-1-heptanol und 5-Methyl-1-heptanol, die je nach den angewandten Herstellungsbedingungen und -verfahren in unterschiedlichen Mengenverhältnissen im Isooctanol vorliegen können. Isooctanol wird üblicherweise durch die Codimerisierung von Propen mit Butenen, vorzugsweise n-Butenen, und anschließende Hydroformylierung des dabei erhaltenen Gemisches aus Heptenisomeren hergestellt. Das bei der Hydroformylierung erhaltene Octanal-Isomerengemisch kann anschließend auf an sich herkömmliche Weise zum Isooctanol hydriert werden.

Die Codimerisierung von Propen mit Butenen zu isomeren Heptenen kann vorteilhaft mit Hilfe des homogenkatalysierten Dimersol®-Verfahrens (Chauvin et al; Chem. Ind.; Mai 1974, S. 375 - 378) erfolgen, bei dem als Katalysator ein löslicher Nickel-Phosphin-Komplex in Gegenwart einer Ethylaluminiumchlor-Verbindung, beispielsweise Ethylaluminiumdichlorid, dient. Als Phosphin-Liganden für den NickelkomplexKatalysator können z. B. Tributylphosphin, Tri-isopropyl-phosphin, Tricyclohexylphosphin und/oder Tribenzylphosphin eingesetzt werden. Die Umsetzung findet bei Temperaturen von 0 bis 80°C statt, wobei vorteilhaft ein Druck eingestellt wird, bei dem die Olefine im flüssigen Reaktionsgemisch gelöst vorliegen (Cornils; Hermann: Applied Homogeneous Catalysis with Organometallic Compounds; 2. Auflage; Bd. 1; S. 254 - 259, Wiley-VCH, Weinheim 2002).

Alternativ zum mit homogen im Reaktionsmedium gelösten Nickelkatalysatoren betriebenen Dimersol®-Verfahren kann die Codimerisierung von Propen mit Butenen auch mit auf einem Träger abgeschiedenen, heterogenen NiO-Katalysatoren durchgeführt werden, wobei ähnliche Hepten-Isomerenverteilungen erhalten werden wie beim homogen katalysierten Verfahren. Solche Katalysatoren werden beispielsweise im sogenannten Octol®-Verfahren (Hydrocarbon Processing, Februar 1986, S. 31 - 33) verwendet, ein gut geeigneter spezifischer Nickel-Heterogenkatalysator zur Olefindimerisierung bzw. Codimerisierung ist z. B. in WO 9514647 offenbart.

Anstelle von Katalysatoren auf Basis von Nickel können auch Brønsted-acide heterogene Katalysatoren zur Codimerisierung von Propen mit Butenen verwendet werden, wobei in der Regel höher verzweigte Heptene als in den Nickel-katalysierten Verfahren erhalten werden. Beispiele von hierfür geeigneten Katalysatoren sind feste Phosphorsäure-Katalysatoren z. B. mit Phosphorsäure imprägnierte Kieselgur oder Diatomeenerde, wie sie vom PolyGas®-Verfahren zur Olefindi- bzw. Oligomerisierung benutzt werden (Chitnis et al; Hydrocarbon Engineering 10, Nr. 6 - Juni 2005). Zur Codimerisierung von Propen und Butenen zu Heptenen sehr gut geeignete Brønsted-acide Katalysatoren sind Zeolithe, deren sich das auf Basis des PolyGas®-Verfahrens weiterentwickelte EMOGAS®-Verfahren bedient.

Das 1-Hepten und die Hepten-Isomerengemische werden nach den vorstehend in Zusammenhang mit der Herstellung von n-Heptanal und Heptanal-Isomerengemische erläuterten bekannten Verfahren mittels Rhodium- oder Kobalt-katalysierter Hydroformylierung, vorzugsweise Kobalt-katalysierter Hydroformylierung, in n-Octanal bzw. Octanal-Isomerengemische überführt. Diese werden anschließend z. B. mittels eines der vorstehend in Zusammenhang mit der n-Heptanol- und Isoheptanol-Herstellung genannten Katalysatoren zu den entsprechenden Octanolen hydriert.

### Nonanol

Im Wesentlichen geradkettiges Nonanol kann durch die Rhodium- oder vorzugsweise Kobaltkatalysierte Hydroformylierung von 1-Octen und nachfolgende Hydrierung des dabei erhaltenen n-Nonanals erhalten werden. Das Ausgangsolefin 1-Octen kann beispielsweise über eine Ethylenoligomerisierung mittels einem homogen im Reaktionsmedium - 1,4-Butandiol - löslichen Nickelkomplexkatalysator mit z. B. Diphenylphosphinoessigsäure oder 2-Diphenylphosphinobenzoesäure als Liganden erhalten werden. Dieses Verfahren ist auch unter der Bezeichnung Shell Higher Olefins Process oder SHOP-Verfahren bekannt (s. Weisermel, Arpe: Industrielle Organische Chemie; 5. Auflage; S. 96; Wiley-VCH, Weinheim 1998).

Bei der im erfindungsgemäßen Verfahren eingesetzte Alkoholkomponente Isononanol handelt es sich nicht um eine einheitliche chemische Verbindung sondern um ein Gemisch aus unterschiedlich verzweigten, isomeren C₉-Alkoholen, die je nach der Art ihrer Herstellung, insbesondere auch der verwendeten Ausgangsstoffe, unterschiedliche Verzweigungsgrade haben können. Im Allgemeinen werden die Isononanole durch Dimerisierung von Butenen zu Isooctengemischen, anschließende Hydroformylierung der Isooctengemische und Hydrierung der dabei erhaltenen Isononanalgemische zu Isononanolgemischen hergestellt, wie in Ullmann's Encyclopedia of Industrial Chemistry, 5. Auflage, Bd. A1, S. 291 - 292, VCH Verlagsgesellschaft GmbH, Weinheim 1995, erläutert.

Als Ausgangsmaterial zur Herstellung der Isononanole können sowohl Isobuten, cis- und trans-2-Buten als auch 1-Buten oder Gemische dieser Butenisomere verwendet werden. Bei der vorwiegend mittels flüssiger, z. B. Schwefel- oder Phosphorsäure, oder fester, z. B. auf Kieselgur, SiO₂ oder Al₂O₃ als Trägermaterial aufgebrachter Phosphorsäure oder Zeolithe, oder Brønsted-Säuren katalysierten Dimerisierung von reinem Isobuten, wird überwiegend das stark verzweigte 2,4,4-Trimethylpenten, auch als Diisobutylen bezeichnet, erhalten, das nach Hydroformylierung und Hydrierung des Aldehyds hochverzweigte Isononanole liefert.

Bevorzugt sind Isononanole mit einem geringeren Verzweigungsgrad. Solche gering verzweigten Isononanol-Gemische werden aus den linearen Butenen 1-Buten, cis- und/oder trans-2-Buten, die gegebenenfalls noch geringere Mengen an Isobuten enthalten können, über den vorstehend beschriebenen Weg der Butendimerisierung, Hydroformylierung des Isooctens und Hydrierung der erhaltenen Isononanal-Gemische hergestellt. Ein bevorzugter Rohstoff ist das sogenannte Raffinat II, das aus dem C₄-Schnitt eines Crackers, beispielsweise eines Steamcrackers, nach Eliminierung von Allenen, Acetylenen und Dienen, insbesondere 1,3-Butadien durch dessen Partialhydrierung zu linearen Butenen oder dessen Abtrennung durch Extraktivdestillation, beispielsweise mittels N-Methylpyrrolidon, und nachfolgender Brønsted-Säure katalysierter Entfernung des darin enthaltenen Isobutens durch dessen Umsetzung mit Methanol oder Isobutanol nach großtechnisch etablierten Verfahren unter Bildung des Kraftstoffadditivs Methyl-tert.-Butylether (MTBE) oder des zur Gewinnung von Rein-Isobuten dienenden Isobutyl-tert.-Butylether, gewonnen wird.

Raffinat II enthält neben 1-Buten und cis- und trans-2-Buten noch n- und iso-Butan und Restmengen von bis zu 5 Gew.-% an Isobuten.

Die Dimerisierung der linearen Butene oder des im Raffinat II enthaltenen Butengemischs kann mittels der gängigen, großtechnisch praktizierten Verfahren, wie sie vorstehend in Zusammenhang mit der Erzeugung von Isoheptengemischen erläutert wurden, beispielsweise mittels heterogener, Brønsted-acider Katalysatoren wie sie im PolyGas®- oder EMOGASO-Verfahren eingesetzt werden, mittels des Dimersol®-Verfahrens unter Verwendung homogen im Reaktionsmedium gelöster Nickel-KomplexKatalysatoren oder mittels heterogener, Nickel(II)oxid-haltiger Katalysatoren nach dem Octol®-Verfahren oder dem Verfahren gemäß WO 9514647 durchgeführt werden. Die dabei erhaltenen Isoocten-Gemische werden nach den vorstehend in Zusammenhang mit der Herstellung von Heptanal-Isomerengemische erläuterten bekannten Verfahren mittels Rhodium- oder Kobalt-katalysierter Hydroformylierung, vorzugsweise Kobalt-katalysierter Hydroformylierung, in Isononanal-Gemische überführt. Diese werden anschließend z. B. mittels eines der vorstehend in Zusammenhang mit der Isoheptanol-Herstellung genannten Katalysatoren zu den geeigneten Isononanolgemischen hydriert.

Die so hergestellten Isononanol-Isomerengemische können über ihren Isoindex charakterisiert werden, der aus dem Verzweigungsgrad der einzelnen, isomeren Isononanolkomponenten im Isononanolgemisch multipliziert mit deren prozentualen Anteil im Isononanolgemisch errechnet werden kann. So tragen z. B. n-Nonanol mit dem Wert 0, Methyloctanole (eine Verzweigung) mit dem Wert 1 und Dimethylheptanole (zwei Verzweigungen) mit dem Wert 2 zum Isoindex eines Isononanolgemisches bei. Je höher die Linearität, desto niedriger ist der Isoindex des betreffenden Isononanolgemisches. Dementsprechend kann der Isoindex eines Isononanolgemisches durch gaschromatographische Auftrennung des Isononanolgemisches in seine einzelnen Isomere und damit einhergehender Quantifizierung von deren prozentualen Mengenanteil im Isononanolgemisch, bestimmt nach Standardmethoden der gaschromatographischen Analyse, ermittelt werden. Zwecks Erhöhung der Flüchtigkeit und Verbesserung der gaschromatographischen Auftrennung der isomeren Nonanole werden diese zweckmäßigerweise vor der gaschromatographischen Analyse mittels Standardmethoden, beispielsweise durch Umsetzung mit N-Methyl-N-trimethylsilyltrifluoracetamid, trimethylsilyliert. Um eine möglichst gute Trennung der einzelnen Komponenten bei der gaschromatographischen Analyse zu erzielen, werden vorzugsweise Kapillarsäulen mit Polydimethylsiloxan als stationärer Phase verwendet. Solche Kapillarsäulen sind im Handel erhältlich und es bedarf lediglich einiger weniger Routineversuche des Fachmanns, um aus dem vielfältigen Angebot des Handels ein optimal für diese Trennaufgabe geeignetes Fabrikat auszuwählen.

Bei den im erfindungsgemäßen Verfahren eingesetzten Isononanolen handelt es sich im Allgemeinen um Isononanole mit einem Isoindex von 0,8 bis 2, vorzugsweise von 1,0 bis 1,8 und besonders bevorzugt von 1,1 bis 1,5 verestert bzw. verethert, die nach den vorstehend genannten Verfahren hergestellt werden können.

Lediglich beispielhaft werden im Folgenden mögliche Zusammensetzungen von Isononanolgemischen angegeben, wie sie im erfindungsgemäßen Verfahren eingesetzt werden können, wobei anzumerken ist, dass die Anteile der im Einzelnen aufgeführten Isomeren im Isononanolgemisch abhängig von der Zusammensetzung des Ausgangsmaterials, beispielsweise Raffinat II, dessen Zusammensetzung an Butenen produktionsbedingt variieren kann, und von Schwankungen in den angewandten Produktionsbedingungen, beispielsweise dem Alter der benutzten Katalysatoren und daran anzupassenden Temperatur- und Druckbedingungen, variieren können.

Beispielsweise kann ein Isononanolgemisch, das durch Kobalt-katalysierte Hydroformylierung und anschließende Hydrierung aus einem unter Verwendung von Raffinat II als Rohstoff mittels des Katalysators und Verfahrens gemäß WO 9514647 erzeugten Isooctengemisches hergestellt wurde, folgende Zusammensetzung haben:
- 1,73 bis 3,73 Gew.-%, bevorzugt 1,93 bis 3,53 Gew.-%, besonders bevorzugt 2,23 bis 3,23 Gew.-% 3-Ethyl-6-methyl-hexanol;
- 0,38 bis 1,38 Gew.-%, bevorzugt 0,48 bis 1,28 Gew.-%, besonders bevorzugt 0,58 bis 1,18 Gew.-% 2,6-Dimethylheptanol;
- 2,78 bis 4,78 Gew.-%, bevorzugt 2,98 bis 4,58 Gew.-%, besonders bevorzugt 3,28 bis 4,28 Gew.-% 3,5-Dimethylheptanol;
- 6,30 bis 16,30 Gew.-%, bevorzugt 7,30 bis 15,30 Gew.-%, besonders bevorzugt 8,30 bis 14,30 Gew.-% 3,6-Dimethylheptanol;
- 5,74 bis 11,74 Gew.-%, bevorzugt 6,24 bis 11,24 Gew.-%, besonders bevorzugt 6,74 bis 10,74 Gew.-% 4,6-Dimethylheptanol;
- 1,64 bis 3,64 Gew.-%, bevorzugt 1,84 bis 3,44 Gew.-%, besonders bevorzugt 2,14 bis 3,14 Gew.-% 3,4,5-Trimethylhexanol;
- 1,47 bis 5,47 Gew.-%, bevorzugt 1,97 bis 4,97 Gew.-%, besonders bevorzugt 2,47 bis 4,47 Gew.-% 3,4,5-Trimethylhexanol, 3-Methyl-4-ethylhexanol und 3-Ethyl-4-methylhexanol;
- 4,00 bis 10,00 Gew.-%, bevorzugt 4,50 bis 9,50 Gew.-%, besonders bevorzugt 5,00 bis 9,00 Gew.-% 3,4-Dimethylheptanol;
- 0,99 bis 2,99 Gew.-%, bevorzugt 1,19 bis 2,79 Gew.-%, besonders bevorzugt 1,49 bis 2,49 Gew.-% 4-Ethyl-5-methylhexanol und 3-Ethylheptanol;
- 2,45 bis 8,45 Gew.-%, bevorzugt 2,95 bis 7,95 Gew.-%, besonders bevorzugt 3,45 bis 7,45 Gew.-% 4,5-Dimethylheptanol und 3-Methyloctanol;
- 1,21 bis 5,21 Gew.-%, bevorzugt 1,71 bis 4,71 Gew.-%, besonders bevorzugt 2,21 bis 4,21 Gew.-% 4,5-Dimethylheptanol;
- 1,55 bis 5,55 Gew.-%, bevorzugt 2,05 bis 5,05 Gew.-%, besonders bevorzugt 2,55 bis 4,55 Gew.-% 5,6-Dimethylheptanol;
- 1,63 bis 3,63 Gew.-%, bevorzugt 1,83 bis 3,43 Gew.-%, besonders bevorzugt 2,13 bis 3,13 Gew.-% 4-Methyloctanol;
- 0,98 bis 2,98 Gew.-%, bevorzugt 1,18 bis 2,78 Gew.-%, besonders bevorzugt 1,48 bis 2,48 Gew.-% 5-Methyloctanol;
- 0,70 bis 2,70 Gew.-%, bevorzugt 0,90 bis 2,50 Gew.-%, besonders bevorzugt 1,20 bis 2,20 Gew.-% 3,6,6-Trimethylhexanol;
- 1,96 bis 3,96 Gew.-%, bevorzugt 2,16 bis 3,76 Gew.-%, besonders bevorzugt 2,46 bis 3,46 Gew.-% 7-Methyloctanol;
- 1,24 bis 3,24 Gew.-%, bevorzugt 1,44 bis 3,04 Gew.-%, besonders bevorzugt 1,74 bis 2,74 Gew.-% 6-Methyloctanol;
- 0,1 bis 3 Gew.-%, bevorzugt 0,2 bis 2 Gew.-%, besonders bevorzugt 0,3 bis 1 Gew.-% n-Nonanol;
- 25 bis 35 Gew.-%, bevorzugt 28 bis 33 Gew.-%, besonders bevorzugt 29 bis 32 Gew.-% sonstige Alkohole mit 9 und 10 Kohlenstoffatomen; mit der Maßgabe, dass die die Gesamtsumme der genannten Komponenten 100 Gew.-% ergibt.

Entsprechend den vorstehenden Ausführungen kann ein Isononanolgemisch, das durch Kobalt-katalysierte Hydroformylierung und anschließende Hydrierung unter Verwendung eines Ethylen-haltigen Butengemisches als Rohstoff mittels des PolyGas®- oder EMOGASO-Verfahrens erzeugten Isooctengemisches hergestellt wurde, im Bereich der folgenden Zusammensetzungen, abhängig von der Rohstoffzusammensetzung und Schwankungen der angewandten Reaktionsbedingungen variieren:
- 6,0 bis 16,0 Gew.-%, bevorzugt 7,0 bis 15,0 Gew.-%, besonders bevorzugt 8,0 bis 14,0 Gew.-% n-Nonanol;
- 12,8 bis 28,8 Gew.-%, bevorzugt 14,8 bis 26,8 Gew.-%, besonders bevorzugt 15,8 bis 25,8 Gew.-% 6-Methyloctanol;
- 12,5 bis 28,8 Gew.-%, bevorzugt 14,5 bis 26,5 Gew.-%, besonders bevorzugt 15,5 bis 25,5 Gew.-% 4-Methyloctanol;
- 3,3 bis 7,3 Gew.-%, bevorzugt 3,8 bis 6,8 Gew.-%, besonders bevorzugt 4,3 bis 6,3 Gew.-% 2-Methyloctanol;
- 5,7 bis 11,7 Gew.-%, bevorzugt 6,3 bis 11,3 Gew.-%, besonders bevorzugt 6,7 bis 10,7 Gew.-% 3-Ethylheptanol;
- 1,9 bis 3,9 Gew.-%, bevorzugt 2,1 bis 3,7 Gew.-%, besonders bevorzugt 2,4 bis 3,4 Gew.-% 2-Ethylheptanol;
- 1,7 bis 3,7 Gew.-%, bevorzugt 1,9 bis 3,5 Gew.-%, besonders bevorzugt 2,2 bis 3,2 Gew.-% 2-Propylhexanol;
- 3,2 bis 9,2 Gew.-%, bevorzugt 3,7 bis 8,7 Gew.-%, besonders bevorzugt 4,2 bis 8,2 Gew.-% 3,5-Dimethylheptanol;
- 6,0 bis 16,0 Gew.-%, bevorzugt 7,0 bis 15,0 Gew.-%, besonders bevorzugt 8,0 bis 14,0 Gew.-% 2,5-Dimethylheptanol;
- 1,8 bis 3,8 Gew.-%, bevorzugt 2,0 bis 3,6 Gew.-%, besonders bevorzugt 2,3 bis 3,3 Gew.-% 2,3-Dimethylheptanol;
- 0,6 bis 2,6 Gew.-%, bevorzugt 0,8 bis 2,4 Gew.-%, besonders bevorzugt 1,1 bis 2,1 Gew.-% 3-Ethyl-4-methylhexanol;
- 2,0 bis 4,0 Gew.-%, bevorzugt 2,2 bis 3,8 Gew.-%, besonders bevorzugt 2,5 bis 3,5 Gew.-% 2-Ethyl-4-methylhexanol;
- 0,5 bis 6,5 Gew.-%, bevorzugt 1,5 bis 6 Gew.-%, besonders bevorzugt 1,5 bis 5,5 Gew.-% sonstige Alkohole mit 9 Kohlenstoffatomen;
mit der Maßgabe, dass sich die Gesamtsumme der genannten Komponenten zu 100 Gew.-% ergibt.

### Decanol

Bei der im erfindungsgemäßen Verfahren eingesetzten Alkoholkomponente Isodecanol handelt es sich nicht um eine einheitliche chemische Verbindung sondern um ein komplexes Gemisch unterschiedlich verzweigter, isomerer Decanole.

Diese werden im Allgemeinen durch die Nickel- oder Brønsted-Säure-katalysierte Trimerisierung von Propylen, beispielsweise nach dem vorstehend erläuterten PolyGas®- oder dem EMOGASO-Verfahren, nachfolgende Hydroformylierung des dabei erhaltenen Isononen-Isomerengemisches mittels homogener Rhodium- oder Kobaltcarbonyl-Katalysatoren, vorzugsweise mittels Kobaltcarbonyl-Katalysatoren und Hydrierung des entstandenen Isodecanal-Isomerengemisches z. B. mittels der vorstehend in Zusammenhang mit der Herstellung von C₇- bis C₉-Alkoholen genannten Katalysatoren und Verfahren (Ullmann's Encyclopedia of Industrial Chemistry; 5. Auflage, Bd. A1, S. 293, VCH Verlagsgesellschaft GmbH, Weinheim 1985) hergestellt. Das so produzierte Isodecanol ist im Allgemeinen stark verzweigt.

Bei dem im erfindungsgemäßen Verfahren eingesetzen 2-Propylheptanol kann es sich um reines 2-Propylheptanol handeln oder um Propylheptanol-Isomerengemische handeln, wie sie im Allgemeinen bei der industriellen Herstellung von 2-Propylheptanol gebildet werden und gemeinhin ebenfalls als 2-Propylheptanol bezeichnet werden.

Reines 2-Propylheptanol kann durch Aldolkondensation von n-Valeraldehyd und nachfolgende Hydrierung des dabei gebildeten 2-Propylheptenals, beispielsweise gemäß US-A 2921089, erhalten werden. Im Allgemeinen enthält kommerziell erhältliches 2-Propylheptanol neben der Hauptkomponente 2-Propylheptanol herstellungsbedingt eines oder mehrere der 2-Propylheptanol-Isomeren 2-Propyl-4-methylhexanol, 2-Propyl-5-methylhexanol, 2-Isopropyl-heptanol, 2-Isopropyl-4-methylhexanol, 2-Isopropyl-5-methylhexanol und/oder 2-Propyl-4,4-dimethylpentanol. Die Anwesenheit anderer Isomere des 2-Propylheptanols, beispielsweise 2-Ethyl-2,4-dimethylhexanol, 2-Ethyl-2-methyl-heptanol und/oder 2-Ethyl-2,5-dimethylhexanol im 2-Propylheptanol ist möglich, aufgrund der geringen Bildungsraten der aldehydischen Vorläufer dieser Isomere im Zuge der Aldolkondensation sind diese, wenn überhaupt, nur in Spurenmengen im 2-Propylheptanol enthalten und spielen für die Weichmachereigenschaften des aus solchen 2-Propyheptanol-Isomerengemischen hergestellten Verbindungen praktisch keine Rolle.

Als Ausgangsmaterial zur Herstellung von 2-Propylheptanol können verschiedenerlei Kohlenwasserstoffquellen benutzt werden, beispielsweise 1-Buten, 2-Buten, Raffinat I - ein aus dem C₄-Schnitt eines Crackers nach Abtrennung von Allenen, Acetylenen und Dienen erhaltenes Alkan/Alken-Gemisch, das neben 1- und 2-Buten noch erhebliche Mengen an Isobuten enthält - oder Raffinat II, das aus Raffinat I durch Abtrennung von Isobuten erhalten wird und als Olefinkomponenten außer 1- und 2-Buten nur noch geringe Anteile an Isobuten enthält. Selbstverständlich können auch Gemische aus Raffinat I und Raffinat II als Rohstoff zur 2-Propylheptanol-Herstellung verwendet werden. Diese Olefine oder Olefingemische können nach an sich herkömmlichen Methoden mit Kobalt- oder Rhodium-Katalysatoren hydroformyliert werden, wobei aus 1-Buten ein Gemisch aus n- und iso-Valeraldehyd - die Bezeichnung iso-Valeraldehyd bezeichnet die Verbindung 2-Methylbutanal - gebildet wird, dessen n/iso-Verhältnis je nach verwendetem Katalysator und Hydroformylierungsbedingungen in relativ weiten Grenzen variieren kann. Beispielsweise wird bei Verwendung eines mit Triphenylphosphin modifizierten homogenen Rhodium-Katalysators (Rh/TPP) aus 1-Buten n- und iso-Valeraldehyd in einem n/iso-Verhältnis von im Allgemeinen 10 : 1 bis 20 : 1 gebildet, wohingegen bei Verwendung von mit Phosphit-Liganden, beispielsweise gemäß US-A 5288918 oder WO 05028407, oder von mit Phosphoamidit-Liganden, beispielsweise gemäß WO 0283695, modifizierten Rhodium-Hydroformylierungskatalysatoren fast ausschließlich n-Valeraldehyd gebildet wird. Während das Rh/TPP-Katalysatorsystem 2-Buten bei der Hydroformylierung nur sehr langsam umsetzt, so dass der größte Teil des 2-Butens aus dem Hydroformylierungsgemisch wieder zurückgewonnen werden kann, gelingt die Hydroformylierung des 2-Butens mit den erwähnten Phosphit-Ligand- oder Phosphoramidit-Ligand-modifizierten Rhodium-Katalysatoren, wobei vorwiegend n-Valeraldehyd gebildet wird. Hingegen wird im olefinischen Rohstoff enthaltenes Isobuten, wenn auch mit unterschiedlicher Geschwindigkeit, von praktisch allen Katalysatorsystemen zu 3-Methylbutanal und je nach Katalysator in geringerem Umfang zu Pivalaldehyd hydroformyliert.

Die je nach verwendeten Ausgangsmaterialien und Katalysatoren erhaltenen C₅-Aldehyde, d. h. n-Valeraldehyd gegebenenfalls im Gemisch mit iso-Valeraldehyd, 3-Methylbutanal und/oder Pivalaldehyd, können vor der Aldolkondensation gewünschtenfalls vollständig oder teilweise destillativ in die Einzelkomponenten aufgetrennt werden, so dass auch hier eine Möglichkeit besteht, die Isomerenzusammensetzung der im erfindungsgemäßen Herstellungsverfahren eingesetzten C₁₀-Alkoholkomponente zu beeinflussen und zu steuern. Desgleichen ist es möglich, das C₅-Aldehydgemisch, wie es bei der Hydroformylierung gebildet wird, ohne die vorherige Abtrennung einzelner Isomere der Aldolkondensation zuzuführen. Bei der Aldolkondensation, die mittels eines basischen Katalysators, wie einer wässrigen Lösung von Natrium- oder Kaliumhydroxid, beispielsweise nach den in EP-A 366089, US-A 4426524 oder US-A 5434313 beschriebenen Verfahren durchgeführt werden kann, entsteht bei Einsatz von n-Valeraldehyd als einziges Kondensationsprodukt 2-Propylheptenal, wohingegen bei Einsatz eines Gemisches isomerer C₅-Aldehyde ein Isomerengemisch aus den Produkten der Homoaldolkondensation gleicher Aldehydmoleküle und der gekreuzten Aldolkondensation unterschiedlicher Valeraldehyd-Isomere geformt wird. Selbstverständlich kann die Aldolkondensation durch die gezielte Umsetzung einzelner Isomere so gesteuert werden, dass überwiegend oder vollständig ein einzelnes Aldolkondensationsisomer gebildet wird. Die betreffenden Aldolkondensationsprodukte können anschließend, üblicherweise nach vorausgegangener, vorzugsweise destillativer Abtrennung aus der Reaktionsmischung und gewünschtenfalls destillativer Reinigung, mit herkömmlichen Hydrierkatalysatoren, beispielsweise den vorstehend zur Hydrierung von Aldehyden genannten, zu den entsprechenden Alkoholen oder Alkoholgemischen hydriert werden.

Im Allgemeinen werden im erfindungsgemäßen Verfahren Gemische des 2-Propylheptanols mit den genannten Propylheptanol-Isomeren eingesetzt, in denen der Gehalt an 2-Propylheptanol mindestens 50 Gew.-%, vorzugsweise 60 bis 98 Gew.-% und besonders bevorzugt 80 bis 95 Gew.-%, insbesondere 85 bis 95 Gew.-% beträgt.

Geeignete Mischungen von 2-Propylheptanol mit den Propylheptanol-Isomeren umfassen beispielsweise solche aus 60 bis 98 Gew.-% 2-Propylheptanol, 1 bis 15 Gew.-% 2-Propyl-4-methyl-hexanol und 0,01 bis 20 Gew.-% 2-Propyl-5-methyl-hexanol und 0,01 bis 24 Gew.-% 2-Isopropylheptanol, wobei die Summe der Anteile der einzelnen Bestandteile 100 Gew.-% nicht überschreitet. Bevorzugt addieren sich die Anteile der einzelnen Bestandteile zu 100 Gew.-%.

Weitere geeignete Mischungen aus 2-Propylheptanol mit den Propylheptanol-Isomeren umfassen beispielsweise solche aus 75 bis 95 Gew.-% 2-Propylheptanol, 2 bis 15 Gew.-% 2-Propyl-4-methyl-hexanol, 1 bis 20 Gew.-% 2-Propyl-5-methyl-hexanol, 0,1 bis 4 Gew.-% 2-Isopropylheptanol, 0,1 bis 2 Gew.-% 2-Isopropyl-4-methylhexanol und 0,1 bis 2 Gew.-% 2-Isopropyl-5-methyl-hexanol, wobei die Summe der Anteile der einzelnen Bestandteile 100 Gew.-% nicht überschreitet. Bevorzugt addieren sich die Anteile der einzelnen Bestandteile zu 100 Gew.-%.

Bevorzugte Mischungen von 2-Propylheptanol mit den Propylheptanol-Isomeren umfassen solche mit 85 bis 95 Gew.-% 2-Propylheptanol, 5 bis 12 Gew.-% 2-Propyl-4-methylhexanol und 0,1 bis 2 Gew.-% 2-Propyl-5-methylhexanol und 0,01 bis 1 Gew.-% 2-Isopropylheptanol, wobei die Summe der Anteile der einzelnen Bestandteile 100 Gew.-% nicht überschreitet. Bevorzugt addieren sich die Anteile der einzelnen Bestandteile zu 100 Gew.-%.

Bei Verwendung der genannten 2-Propylheptanol-Isomerengemische anstelle von reinem 2-Propylheptanol entspricht die Isomerenzusammensetzung der Alkylestergruppen der Produkte praktisch der Zusammensetzung der zur Veresterung verwendeten Propylheptanol-Isomerengemische.

### Undecanol

Die im erfindungsgemäßen Verfahren eingesetzten Undecanole können geradkettig oder verzweigt sein oder aus Gemischen geradkettiger und verzweigter Undecanole zusammengesetzt sein. Bevorzugt werden Gemische aus verzweigten Undecanolen, auch als Isoundecanol bezeichnet, als Alkoholkomponente verwendet.

Im Wesentlichen geradkettiges Undecanol kann durch die Rhodium- oder vorzugsweise Kobaltkatalysierte Hydroformylierung von 1-Decen und nachfolgende Hydrierung des dabei erhaltenen n-Undecanals erhalten werden. Das Ausgangsolefin 1-Decen wird über das zuvor bei der Herstellung von 1-Octen erwähnte SHOP-Verfahren hergestellt.

Zur Herstellung verzweigten Isoundecanols kann das im SHOP-Verfahren erhaltene 1-Decen einer Skelettisomerisierung z. B. mittels acider zeolithischer Molekularsiebe, wie in WO 9823566 beschrieben, unterzogen werden, wobei sich Gemische aus isomeren Decenen bilden, deren Rhodium- oder vorzugsweise Kobalt-katalysierte Hydroformylierung und nachfolgende Hydrierung der erhaltenen Isoundecanal-Gemische zu dem zur Herstellung der erfindungsgemäßen Verbindungen verwendeten Isoundecanols führt. Die Hydroformylierung von 1-Decen oder Isodecen-Gemischen mittels Rhodium- oder Kobalt-Katalyse kann wie zuvor in Zusammenhang mit der Synthese von C₇- bis C₁₀-Alkoholen beschrieben erfolgen. Entsprechendes gilt für die Hydrierung von n-Undecanal oder Isoundecanal-Gemischen zu n-Undecanol bzw. Isoundecanol.

Nach destillativer Reinigung des Austrags der Hydrierung können die so erhaltenen C₇-bis C₁₁-Alkylalkohole bzw. deren Gemische, in dem erfindungsgemäßen Verfahren verwendet werden.

### Dodecanol

Im Wesentlichen geradkettiges Dodecanol kann in vorteilhafter Weise über das Alfol®- oder Epal®-Verfahren gewonnen werden. Diese Verfahren beinhalten die Oxidation und Hydrolyse geradkettiger Trialkylaluminium-Verbindungen, welche ausgehend von Triethylaluminium schrittweise über mehrere Ethylierungsreaktionen unter Verwendung von Ziegler-Natta-Katalysatoren aufgebaut werden. Aus den daraus resultierenden Gemischen weitgehend geradkettiger Alkylalkohole unterschiedlicher Kettenlänge kann nach dem destillativen Austrag der C₁₂-Alkylalkohol-Fraktion das gewünschte n-Dodecanol erhalten werden.

Alternativ kann n-Dodecanol auch durch Hydrogenierung natürlicher Fettsäuremethylester, beispielsweise aus Kokosnussöl, hergestellt werden.

Verzweigtes Isododecanol kann analog zu den zuvor beschriebenen Verfahren zur Codimerisierung und/oder Oligomerisierung von Olefinen mit nachfolgender Hydroformylierung und Hydrierung der Isoundecen-Gemische erhalten werden. Nach destillativer Reinigung des Austrags der Hydrierung können die so erhaltenen Isododecanole bzw. deren Gemische, wie vorstehend beschrieben, in dem erfindungsgemäßen Verfahren verwendet werden.

In einer bevorzugten Ausführungsform des erfindungsgemäßen Verfahrens sind die Alkoxyalkanole ausgewählt unter Verbindungen der allgemeinen Formel R²-[O-X]ₙ-OH worin
- R²: ausgewählt ist unter unverzweigten C₁-C₉-Alkylresten und verzweigten C₃-C₉-Alkylresten,
- X: für eine unverzweigte C₂-C₃-Alkylengruppe oder eine verzweigte C₃-C₄-Alkylengruppe steht und
- n: die Werte 1 oder 2 aufweist.

Besonders bevorzugt ist der Alkohol R²-[O-X]ₙ-OH ausgewählt unter 2-Butoxyethanol, 2-(2-Butoxyethoxy)ethanol, 1-Methoxy-2-propanol, 3-Methoxypropanol oder Mischungen davon.

Die in dem erfindungsgemäßen Verfahren eingesetzten Carbonsäuren und/oder Carbonsäureanhydride sind ausgewählt unter aromatischen Mono-, Di-, Tri- oder Tetracarbonsäuren, aliphatischen Mono- und Dicarbonsäuren, Hydroxycarbonsäuren, alicyclischen Mono-, Di-, Tri- und Tetracarbonsäuren, heterocyclischen Dicarbonsäuren, den Anhydriden der zuvor genannten Carbonsäuren und Mischungen davon.

Bei den im erfindungsgemäßen Verfahren eingesetzten aromatischen Mono-, Di-, Tri- oder Tetracarbonsäuren und deren Anhydride handelt es sich beispielsweise um Benzoesäure, Benzoesäureanhydrid, Phthalsäure, Phthalsäureanhydrid, Isophthalsäure, Terephthalsäure, Trimellitsäure, Trimellitsäureanhydrid, Pyromellitsäure und Pyromellitsäuredianhydrid.

Bei den im erfindungsgemäßen Verfahren eingesetzten aliphatischen Mono- und Dicarbonsäuren handelt es sich beispielsweise um gesättigte Mono- und Dicarbonsäuren wie Essigsäure, Buttersäure, Valeriansäure, Bernsteinsäure, Adipinsäure oder Sebacinsäure, um ungesättigte Mono- und Dicarbonsäuren wie Acrylsäure, Maleinsäure oder der Fumarsäure sowie gegebenenfalls um die Anhydride der zuvor genannten Carbonsäuren.

Bei den im erfindungsgemäßen Verfahren eingesetzten Hydroxycarbonsäuren handelt es sich beispielsweise um Glycolsäure, Milchsäure, Äpfelsäure, Weinsäure oder Citronensäure sowie gegebenenfalls um deren Anhydride.

Bei den im erfindungsgemäßen Verfahren eingesetzten alicyclischen Mono-, Di-, Tri- und Tetracarbonsäuren handelt es sich beispielsweise um die kernhydrierten Derivate der zuvor genannten aromatischen Mono-, Di-, Tri- oder Tetracarbonsäuren, wie Cyclohexancarbonsäure, 1,2-Cyclohexandicarbonsäure, 1,3-Cyclohexandicarbonsäure, 1,4-Cyclohexandicarbonsäure, 1,2,4-Cyclohexantricarbonsäure oder 1,2,4,5-Cyclohexantetracarbonsäure sowie gegebenenfalls um deren Anhydride.

Bei den im erfindungsgemäßen Verfahren eingesetzten heterocyclischen Dicarbonsäuren handelt es sich beispielsweise um 2,5-Furandicarbonsäure oder 2,5-Tetrahydrofurandicarbonsäure.

In einer bevorzugten Ausführungsform des erfindungsgemäßen Verfahrens ist die Carbonsäure und/oder das Carbonsäureanhydrid ausgewählt unter Essigsäure, Essigsäureanhydrid, Benzoesäure, Benzoesäureanhydrid, Phthalsäure, Phthalsäureanhydrid, Isophthalsäure, Terephthalsäure, Trimellitsäure, Trimellitsäureanhydrid, Pyromellitsäure und Pyromellitsäuredianhydrid. Besonders bevorzugt sind die Carbonsäure und/oder das Carbonsäureanhydrid ausgewählt unter Essigsäure, Essigsäureanhydrid, Benzoesäure, Benzoesäureanhydrid, Terephthalsäure, Trimellitsäure, Trimellitsäureanhydrid. Insbesondere wird in dem erfindungsgemäßen Verfahren als Carbonsäure bzw. Carbonsäureanhydrid Essigsäure, Essigsäureanhydrid oder Terephthalsäure eingesetzt.

### Weichmacheranwendung

In Kunststoffen, bei denen die optischen Eigenschaften im Vordergrund stehen, ist es in der Regel wünschenswert, dass die zu deren Herstellung eingesetzten Weichmacher eine geringe Eigenfärbung, d. h. eine niedrige Farbzahl, aufweisen.

Die mit dem erfindungsgemäßen Verfahren hergestellten Carbonsäureester zeichnen sich insbesondere durch eine niedrige Farbzahl aus. Sie eignen sich somit in vorteilhafter Weise für den Einsatz als Weichmacher oder in Weichmachern für thermopastische Polymere und Elastomere.

Darüberhinaus weisen die mit dem erfindungsgemäßen Verfahren hergestellten Carbonsäureester, bedingt durch den Einsatz hochreiner Methansulphonsäure (Lutropur ® MSA oder Lutropur ® MSA 100) als Katalysator, einen geringen Gesamtchlorgehalt als auch einen geringen Sulfatgehalt auf.

Die mit dem erfindungsgemäßen Verfahren hergestellten Carbonsäureester lassen sich in der Regel in allen thermoplastisch verarbeitbaren Polymeren einsetzen, zu deren Herstellung Weichmacher zum Einsatz kommen. Bevorzugt sind diese thermoplastischen Polymere ausgewählt unter Polyvinylchlorid (PVC), Polyvinylbutyral (PVB), Homo- und Copolymere von Vinylacetat, Homo- und Copolymere von Styrol, Polyacrylate, thermoplastische Polyurethane (TPU) oder Polysulfide und Mischungen davon.

Die mit dem erfindungsgemäßen Verfahren hergestellten Carbonsäureester können zudem bei der Herstellung von Elastomeren eingesetzt werden. Bevorzugt handelt es sich dabei um natürlichen Kautschuk (NR) oder auf synthetischem Wege hergestellte Kautschuke, wie beispielsweise Polyisopren-Kautschuk (IR), Styrol-Butadien-Kautschuk (SBR), Butadien-Kautschuk (BR), Nitril-Butadien-Kautschuk (NBR) oder Chloropren-Kautschuk (CR).

Die Erfindung wird anhand der im Folgenden beschriebenen Figuren und der Beispiele näher erläutert. Dabei sollen die Figuren und Beispiele nicht als einschränkend für die Erfindung verstanden werden.

In den nachfolgenden Beispielen bzw. den Figuren werden folgende Abkürzungen verwendet:
MSA steht für Methansulfonsäure,
PTSA steht für para-Toluolsulfonsäure,
MSTFA steht für N-Methyl-N-(trimethylsilyl)trifluoroacetamid,
DOTP steht für Bis(2-ethylhexyl)terephthalat (Dioctylterephthalat),
DINP steht für Diisononylphthalat,
TOTM steht für Tris(2-ethylhexyl)trimellitat,
APHA steht für American Public Health Association,
OiPr steht für Isopropanolat.

### FIGURENBESCHREIBUNG

### Figur 1:

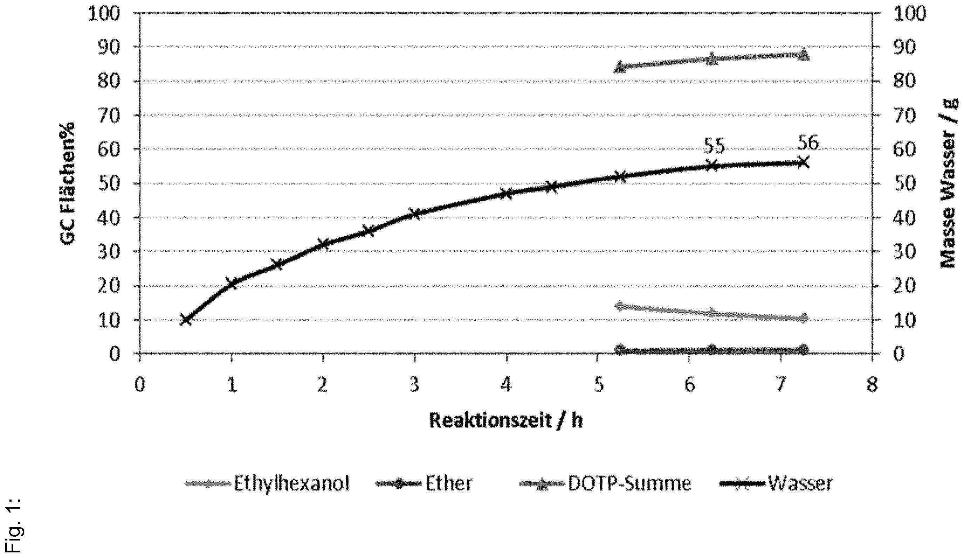

Figur 1 zeigt den Verlauf der MSA katalysierten Veresterungsreaktion von Terephthalsäure und 2-Ethylhexanol (Beispiel 1) anhand der gaschromatographisch ermittelten prozentualen Anteile des Reaktionsproduktes DOTP, des Einsatzstoffes 2-Ethylhexanol sowie des Nebenproduktes Di(2-ethylhexyl)ether in der Reaktionsmischung. Zusätzlich ist die Menge an abgeschiedenem Wasser angegeben.

### BEISPIELE

### I) Analytische Untersuchungen:

### I.a Gaschromatographische Untersuchung:

Für gaschromatographische Untersuchungen wurden die Proben mit einem Überschuss MSTFA (N-Methyl-N-(trimethylsilyl)trifluoroacetamid) versetzt und für 30 min auf 100 °C erhitzt, sodass alle aciden Protonen in die entsprechenden Trimethylsilylgruppen überführt waren. Nach dem Abkühlen wurden die Proben mit N,N-Dimethylformamid (DMF) verdünnt.

Angaben zum gaschromatographisches Trennsystem und zur Trennmethode:
Messgerät: Agilent 6890 Series
Injektor: Split/Splitless mit Split Liner siltec-deactivated (Restec # 20713-214.5)
Säule: Optima 1 (Länge= 25 m, Innendurchmesser = 0,25 mm, Außendurchmesser = 0,40 mm, Filmdicke 0,25 µm) der Firma Macherey & Nagel
Detektor: FID mit 300 ml/min Luft, 30 ml/min Wasserstoff und 30 ml/min Make-Up-Gas (Stickstoff)
Trägergas: Stickstoff
Fluss: 0,7 ml/min bei 8,3 PSI (bei einer Ofentemperatur von 80 °C)
Split: 1:36, Splitflow: 28 ml/min, Septum purge 2,0 ml/min (bei einer Ofentemperatur von 80° C)
Injektortemperatur: 340 °C
Injektionsvolumen: 1 µl
Detektortemperatur: 320 °C
Temperaturprogramm:

| | |
|---|---|
| Start: | 120 °C |
| Verweildauer 1: | 0 min |
| Temperaturrampe 1: | 20 °C/min |
| Endtemperatur 1: | 350 °C |
| Verweildauer 2: | 5 min |
| Gesamtlaufzeit: | 16,5 min |

Wenn die Proben Hochsieder enthalten, kann die Verweildauer 2 alternativ auf 30 min gestellt werden. Die Gesamtlaufzeit erhöht sich dann auf 41,5 min.

Auswertung: Empower-3-Software nach Flächen-%

**Retentionszeiten:**

| | |
|---|---|
| DOTP (Peak 1) | 10,456 min (Hauptpeak) |
| DOTP (Peak 2) | 10,202 min (Isomer von Peak 1) |
| 2-Ethyl-1-Hexanol-MSTFA | 2,87 min |
| 2-Ethyl-1-Hexylmesylat | 4,44 min |
| Terephthalsäure-MSTFA | 6,39 min |
| Monoester-MSTFA | 8,52 min |
| Ethylhexanol-di-Ether | 4,89 min |

### I.b Bestimmung der Säurezahl:

Die Bestimmung der Säurezahl, angegeben in mg KOH/g Probe, erfolgt in Propanol durch potentiometrische Titration mit 0,1 mol/L Tetrabutylammoniumhydroxid-Maßlösung. Die Bestimmung wird auf Geräte und Elektroden der Firma Metrohm durchgeführt.

### I.c Bestimmung der Hazen-Farbzahl nach APHA:

Die Messung der Hazen-Farbzahl erfolgt in Anlehnung an die DIN/EN/ISO 6271-2 (März 2005) in Substanz gegen Wasser als Referenz. Zur Messung werden Rundküvetten mit einem Durchmesser von 11 mm verwendet. Als Messgerät kann z. B. ein Photometer Dr. Lange LICO 400 eingesetzt werden.

### II) Herstellungsbeispiele:

### Beispiel 1:

Synthese von DOTP aus Terephthalsäure und 2-Ethylhexanol mit MSA als Katalysator.

In einem 1.6 L Doppelmantel-Rührkesselreaktor, der über einen programmierbaren Thermostat geheizt werden kann und mit Ankerrührer, Wasserabscheider in Jennewein-Bauart, Kondensator, Stickstoffeinlass und Anschluss für eine Vakuumpumpe ausgestattet ist, wurden Terephthalsäure (249 g, 1,50 mol), 2-Ethylhexanol (469 g, 3,60 mol) und Methansulfonsäure (Lutropur MSA, BASF, 6,13 g einer ca. 70 Gew.-% wässrigen Lösung, 0,045 mol) vorgelegt und der Reaktor mit Stickstoff inertisiert. Der Stickstoffstrom durch die Apparatur wurde auf 2 - 4 L h⁻¹ eingestellt und das Reaktionsgemisch auf 180 °C erhitzt, wobei sich ein azeotropes Gemisch aus Wasser und 2-Ethylhexanol bildete, das im Kondensator verflüssigt und in den Wasserabscheider überführt wurde. Nach der Phasentrennung wurde die organische Phase in den Reaktor zurückgeführt, während die wässrige Phase verworfen wurde. Die ausgeschiedene Menge Wasser wurde gewogen und zur Überwachung der Reaktion verwendet. Um einen konstanten Destillatstrom zu gewährleisten und die Reaktion zu vollem Umsatz zu führen wurde die Temperatur innerhalb von 4 h stufenweise auf 200 °C und schließlich innerhalb von 2 h auf 215 °C erhöht. Dort wurde die Temperatur so lange gehalten, bis das Reaktionsgemisch als klare Lösung vorlag und die berechnete Menge Wasser aus der Reaktion und der eingesetzten Methansulfonsäure gesammelt war (56 g). Die Reaktionszeit betrug 7,25 h. Zusätzlich wurde das Reaktionsgemisch gaschromatographisch untersucht. Nach dem Abkühlen auf Raumtemperatur wurde die Säurezahl des Ansatzes nach den bekannten Methoden bestimmt und der Ansatz mit 150 mL 1,2 % NaOH (50 % Überschuss bezogen auf die ermittelte Säurezahl) alkalisiert. Nach dem Trennen der Phasen wurde der Ansatz mit Wasser neutral gewaschen und der Überschuss 2-Ethylhexanol sowie alle weiteren Verbindungen mit einem Siedepunkt unterhalb des Siedepunktes von DOTP im Vakuum abgezogen (205 °C, 8 mbar). Das so erhaltene Produkt wurde über eine Druckfilternutsche filtriert. Reaktionszeit: 7,25 h. Ausbeute: 91 %. GC-Gehalt: 98,73 % DOTP (Flächen%). Farbzahl (APHA, Hazen): 12.

### Beispiel 2:

Synthese von DOTP aus Terephthalsäure und 2-Ethylhexanol mit MSA als Katalysator unter reduziertem Druck.

Die Reaktion wurde wie in Beispiel 1 beschrieben durchgeführt. Allerdings wurde der Druck während der Reaktion von 750 mbar auf 400 mbar reduziert und die Temperatur bei 140 - 180 °C gehalten. Reaktionszeit: 8 h. Ausbeute: 96 %. GC-Gehalt: 97,27 % DOTP (Flächen%).

### Beispiel 3:

Synthese von Diisononylphthalat (DINP) aus Phthalsäureanhydrid und Isononanol mit MSA als Katalysator.

In einem 1,6 L Doppelmantel-Rührkesselreaktor, der über einen programmierbaren Thermostat geheizt werden kann und mit Ankerrührer, Wasserabscheider in Jennewein-Bauart, Kondensator, Stickstoffeinlass und Anschluss für eine Vakuumpumpe ausgestattet ist, wurden Phthalsäureanhydrid (224 g, 1,50 mol), Isononanol (Nonanol N, BASF SE) (519 g, 3,60 mol) und Methansulfonsäure (Lutropur MSA, BASF, 3,07 g einer ca. 70 Gew.-% wässrigen Lösung, 0.023 mol) vorgelegt und der Reaktor mit Stickstoff inertisiert. Der Stickstoffstrom durch die Apparatur wurde auf 2 - 4 L h⁻¹ eingestellt und das Reaktionsgemisch auf 170 °C erhitzt, wobei sich ein azeotropes Gemisch aus Wasser und Isononanol bildete, das im Kondensator verflüssigt und in den Wasserabscheider überführt wurde. Nach der Phasentrennung wurde die organische Phase in den Reaktor zurückgeführt, während die wässrige Phase verworfen wurde. Die ausgeschiedene Menge Wasser wurde gewogen und zur Überwachung des Reaktionsverlaufs verwendet. Um einen konstanten Destillatstrom zu gewährleisten und die Reaktion zu vollem Umsatz zu führen wurde die Temperatur innerhalb von 2 h stufenweise auf 217 °C erhöht. Nach 2,5 Stunden war die berechnete Menge Wasser ausgeschieden und das Reaktionsgemisch wurde auf Raumtemperatur abgekühlt. Die Säurezahl des Ansatzes wurde nach den bekannten Methoden bestimmt und der Ansatz mit 150 mL 1,2 % NaOH (50 % Überschuss bezogen auf die ermittelte Säurezahl) alkalisiert. Nach dem Trennen der Phasen wurde der Ansatz mit Wasser neutral gewaschen und der Überschuss Isononanol sowie alle weiteren Verbindungen mit einem Siedepunkt unterhalb des Siedepunktes von DINP im Vakuum abgezogen (231 °C, 8 mbar). Das so erhaltene Produkt wurde über eine Druckfilternutsche filtriert. Reaktionszeit: 2,5 h. Ausbeute: 92,8 %. GC-Gehalt: 99,65 % DINP (Flächen%). Farbzahl (APHA, Hazen): 22. Säurezahl: 0,1 mg KOH/g.

### Beispiel 4:

Synthese von Tris(2-ethylhexyl)trimellitat (TOTM) aus Trimellitsäureanhydrid und 2-Ethylhexanol mit MSA als Katalysator.

In einem 1,6 L Doppelmantel-Rührkesselreaktor, der über einen programmierbaren Thermostat geheizt werden kann und mit Ankerrührer, Wasserabscheider in Jennewein-Bauart, Kondensator, Stickstoffeinlass und Anschluss für eine Vakuumpumpe ausgestattet ist, wurden Trimellitsäureanhydrid (252,2 g, 1,31 mol), 2-Ethylhexanol (564 g, 4,33 mol) und Methansulfonsäure (Lutropur MSA, BASF, 2,68 g einer ca. 70 Gew.-% wässrigen Lösung, 0,020 mol) vorgelegt und der Reaktor mit Stickstoff inertisiert. Der Stickstoffstrom durch die Apparatur wurde auf 2 - 4 L h⁻¹ eingestellt und das Reaktionsgemisch auf 180 °C erhitzt, wobei sich ein azeotropes Gemisch aus Wasser und 2-Ethylhexanol bildete, das im Kondensator verflüssigt und in den Wasserabscheider überführt wurde. Nach der Phasentrennung wurde die organische Phase in den Reaktor zurückgeführt, während die wässrige Phase verworfen wurde. Die ausgeschiedene Menge Wasser wurde gewogen und zur Überwachung der Reaktion verwendet. Um einen konstanten Destillatstrom zu gewährleisten und die Reaktion zu vollem Umsatz zu führen wurde die Temperatur innerhalb von 3 h stufenweise auf 208 °C erhöht. Anschließend wurde die Temperatur so lange gehalten, bis die berechnete Menge Wasser aus der Reaktion und der eingesetzten Methansulfonsäure gesammelt war (49 g). Die Reaktionszeit betrug 3 h. Nach dem Abkühlen auf Raumtemperatur wurde die Säurezahl des Ansatzes nach den bekannten Methoden bestimmt und der Ansatz mit 1,80 g Natriumhydrogencarbonat (50 % Überschuss bezogen auf die ermittelte Säurezahl) alkalisiert. Der Überschuss 2-Ethylhexanol sowie alle weiteren Verbindungen mit einem Siedepunkt unterhalb des Siedepunktes von TOTM wurden im Vakuum abgezogen (197 °C, 6 mbar). Das so erhaltene Produkt wurde über eine Druckfilternutsche filtriert. Reaktionszeit: 3 h. Ausbeute: 93 %. GC-Gehalt: 97,40 % TOTM (Flächen%). Farbzahl (APHA, Hazen): 38.

### Beispiel 5 (nicht erfindungsgemäß):

Synthese von DOTP aus Terephthalsäure und 2-Ethylhexanol mit PTSA als Katalysator.

DOTP wurde nach dem in Beispiel 1 beschriebenen Verfahren hergestellt. Allerdings wurde statt Methansulfonsäure PTSA (8,56 g, 0,045 mol) als Katalysator verwendet (p-Toluolsulfonsäure Monohydrat der Firma Sigma-Aldrich, ACS reagent grade, ≥98,5 %, <0,3 % SO₄²⁻). Reaktionszeit: 8 h. Ausbeute: 91 %. GC-Gehalt: 98,09 % DOTP (Flächen%). Farbzahl (APHA, Hazen): 24.

### Vergleichsbeispiel V1:

Synthese von DOTP aus Terephthalsäure und 2-Ethylhexanol mit MSA als Katalysator, ohne Stickstoff.

DOTP wurde nach dem in Beispiel 1 beschriebenen Verfahren hergestellt. Allerdings wurde auf das Durchleiten von Stickstoff verzichtet. Reaktionszeit: 14 h. Ausbeute: 91 %. GC-Gehalt: 99 % DOTP (Flächen%). Farbzahl (APHA, Hazen): 578.

### Vergleichsbeispiel V2:

Synthese von DOTP aus Terephthalsäure und 2-Ethylhexanol mit Schwefelsäure als Katalysator.

DOTP wurde nach dem in Beispiel 1 beschriebenen Verfahren hergestellt. Allerdings wurde statt Methansulfonsäure Schwefelsäure (2,22 g, 0,023 mol) als Katalysator verwendet. Die Temperatur betrug am Ende der Reaktion 236 °C. Reaktionszeit: 8 h. Ausbeute: 88 %. GC-Gehalt: 99,37 % DOTP (Flächen%). Die Farbzahl war zu hoch, um mit der Hazen-Skala erfasst zu werden. Iodfarbzahl: 8,6.

### Vergleichsbeispiel V3:

Synthese von DOTP aus Terephthalsäure und 2-Ethylhexanol mit Ti(OiPr)₄ als Katalysator.

DOTP wurde nach dem in Beispiel 1 beschriebenen Verfahren hergestellt. Allerdings wurde statt Methansulfonsäure Ti(OiPr)₄ (2,22 g, 0,023 mol) als Katalysator verwendet. Die Temperatur betrug am Ende der Reaktion 233 °C. Reaktionszeit: 18 h. Ausbeute: 82 %. GC-Gehalt: 97,70 % DOTP (Flächen%). Farbzahl (APHA, Hazen): 25.

### Beispiel 6 (nicht erfindungsgemäß):

Synthese von TOTM aus Trimellitsäureanhydrid und 2-Ethylhexanol mit PTSA als Katalysator.

TOTM wurde nach dem in Beispiel 4 beschriebenen Verfahren hergestellt. Allerdings wurde statt Methansulfonsäure PTSA (3,88 g, 0,02 mol) als Katalysator verwendet (p-Toluolsulfonsäure Monohydrat der Firma Sigma-Aldrich, ACS reagent grade, ≥98,5 %, <0,3 % SO₄²⁻). Die Temperatur betrug am Ende der Reaktion 207 °C. Reaktionszeit: 4 h. Ausbeute: 91 %. GC-Gehalt: 96,99 % TOTM (Flächen%). Farbzahl (APHA, Hazen): 104.

### Vergleichsbeispiel V4:

Synthese von TOTM aus Trimellitsäureanhydrid und 2-Ethylhexanol mit Ti(OiPr)₄ als Katalysator.

TOTM wurde nach dem in Beispiel 4 beschriebenen Verfahren hergestellt. Allerdings wurde statt Methansulfonsäure Ti(OiPr)₄ (1,11 g, 0,004 mol) als Katalysator verwendet. Die Temperatur betrug am Ende der Reaktion 217 °C. Reaktionszeit: 6 h. Ausbeute: 90 %. GC-Gehalt: 96,93 % TOTM (Flächen%). Farbzahl (APHA, Hazen): 187.

### Beispiel 7 (nicht erfindungsgemäß):

Synthese von DINP aus Phthalsäureanhydrid und Isononanol (Nonanol N, BASF SE) mit PTSA als Katalysator (p-Toluolsulfonsäure Monohydrat der Firma Sigma-Aldrich, ACS reagent grade, ≥98,5 %, <0,3 % SO₄²⁻).

DINP wurde nach dem in Beispiel 3 beschriebenen Verfahren aus 224 g (1,50 mol) Phthalsäureanhydrid und 476 g Nonanol N (3,30 mol) hergestellt. Allerdings wurde statt Methansulfonsäure PTSA (4,37 g, 0,023 mol) als Katalysator verwendet. Die Temperatur betrug am Ende der Reaktion 217 °C.

Reaktionszeit: 4 h. Ausbeute: 94 %. GC-Gehalt: 99,32 % DINP (Flächen%). Farbzahl (APHA, Hazen): 38.

### Vergleichsbeispiel V5:

Synthese von DOTP aus Terephthalsäure und 2-Ethylhexanol unter Verwendung von MSA mit einem hohen Gesamtchlor- und Sulfatgehalt.

DOTP wurde wie in Beispiel 1 dargestellt. Allerdings wurde MSA mit einem hohen Gesamtchlor- und Sulfatgehalt als Katalysator verwendet. Nach der Aufarbeitung betrug die Farbzahl 57 (APHA, Hazen) und der Ansatz wurde nicht weiter charakterisiert.

### Vergleichsbeispiel V6:

Synthese von DOTP aus Terephthalsäure und 2-Ethylhexanol unter Verwendung von PTSA mit einem hohen Sulfatgehalt.

DOTP wurde wie in Beispiel 5 dargestellt. Allerdings wurde PTSA mit einem hohen Sulfatgehalt als Katalysator verwendet. Nach der Aufarbeitung betrug die Farbzahl 44 (APHA, Hazen) und der Ansatz wurde nicht weiter charakterisiert.

### III) Vergleichsbeispiele zur Verfärbung von Veresterungsprodukten in Abhängigkeit der Qualität des verwendeten Veresterungskatalysators an einem Modellsystem auf Basis von Ölsäure und ohne Zuführung eines inerten Gases:

80 g einer equimolaren Mischung aus Ölsäure und 2-Ethylhexanol wurden in 100 ml Laborglasflaschen eingewogen und mit 1 Gew.-% Säure (MSA und Schwefelsäure) bzw. 2 Gew.-% PTSA (aufgrund der in etwa doppelten molaren Masse von 190 g/mol versus 96 g/mol bei MSA) versetzt. Die verschiedenen Flaschen wurden in einen 15-fach Heizrührblock gestellt und bei 150°C über einen Zeitraum von 24 h gerührt. Anschließend wurde von jeder Mischung eine Probe entnommen und die Hazen-Farbzahl bestimmt:

| Beispiel | Katalysator | Sulfatgehalt [%] | Hazen Farbzahl [APHA] |
|---|---|---|---|
| 1 | MSA (A), Lutropur® MSA100 | < 0,005 % (< 50 ppm) | 344 |
| 2 | MSA (B) | 0,02 % (200 ppm) | 528 |
| 3 | PTSA (A), fest | 0,19 % | 542 |
| 4 | PTSA (B), 65%ige Lösung | 0,32 % | 866 |
| 5 | PTSA (C), fest | 10 % | > 1000 |
| 6 | Schwefelsäure | 96 % | > 1000 |

Es zeigte sich, dass mit den eingesetzten sauren Katalysatoren mit hohem Sulfatgehalt schlechte Farbzahlen erzielt werden.

## Patentansprüche

1. Verfahren zur Herstellung von Carbonsäureestern, bei dem man in einem aus einem oder mehreren Reaktoren bestehenden Reaktionssystem ein Reaktionsgemisch umsetzt, das wenigstens eine Carbonsäure und/oder wenigstens ein Carbonsäureanhydrid und wenigstens einen Alkohol R¹-OH und/oder wenigstens einen Alkohol R²-[O-X]ₙ-OH umfasst, worin
R¹ ausgewählt ist unter unverzweigten und verzweigten C₅-C₁₃-Alkylresten und C₅-C₆-Cycloalkylresten, wobei die Cycloalkylreste unsubstituiert sind oder durch wenigstens einen C₁-C₁₀-Alkylrest substituiert sein können,
R² ausgewählt ist unter unverzweigten C₁-C₁₃-Alkylresten und verzweigten C₃-C₁₃-Alkylresten,
X für eine unverzweigte C₂-C₅-Alkylengruppe oder verzweigte C₃-C₅-Alkylengruppe steht und
n die Werte 1, 2 oder 3 aufweist,
mit der Maßgabe, dass die Umsetzung
- in Gegenwart wenigstens eines Katalysators, der ausgewählt ist unter Methansulfonsäure, wobei die Methansulfonsäure einen Sulfatgehalt von höchstens 50 ppm aufweist,
- unter Zuführung eines unter den Reaktionsbedingungen inerten Gases zum Reaktionssystem,
- bei einer Temperatur des Reaktionsgemischs von 125 °C bis 240 °C und
- unter destillativer Abtrennung wenigstens eines Teils des während der Reaktion gebildeten Wassers in Form einer azeotropen Mischung mit dem eingesetzten Alkohol R¹-OH und/oder R²-[O-X]ₙ-OH,
erfolgt, wobei der abdestillierte Alkohol R¹-OH und/oder R²-[O-X]ₙ-OH zumindest teilweise in das Reaktionssystem zurückgeführt wird.

2. Verfahren nach Anspruch 1, wobei man das inerte Gas in wenigstens einen der Reaktoren unterhalb der Flüssigkeitsoberfläche des Reaktionsgemisches einführt und das Reaktionsgemisch mit dem inerten Gas durchperlt.

3. Verfahren nach einem der vorhergehenden Ansprüche, wobei das Reaktionssystem eine Kaskade aus wenigstens zwei Reaktoren umfasst und man das inerte Gas zumindest dem ersten Reaktor der Kaskade zuführt.

4. Verfahren nach einem der vorhergehenden Ansprüche, wobei die Umsetzung in kontinuierlicher Fahrweise durchgeführt wird.

5. Verfahren nach einem der vorangegangenen Ansprüche, wobei der Rest R¹ ausgewählt ist unter n-Octyl, 2-Ethylhexyl, n-Nonyl, Isononyl, Isodecyl, 2-Propylheptyl, n-Undecyl, Isoundecyl.

6. Verfahren nach einem der vorangegangenen Ansprüche, wobei als Alkohol R¹-OH 2-Ethylhexanol eingesetzt wird.

7. Verfahren nach einem der vorangegangenen Ansprüche, wobei in der Formel
R²-[O-X]ₙ-OH
R² ausgewählt ist unter unverzweigten C₁-C₉-Alkylresten und verzweigten C₃-C₉-Alkylresten,
X für eine unverzweigte C₂-C₃-Alkylengruppe oder eine verzweigte C₃-C₄-Alkylengruppe steht und
n die Werte 1 oder 2 aufweist.

8. Verfahren nach einem der vorangegangenen Ansprüche, wobei als Alkohol R²-[O-X]ₙ-OH 2-Butoxyethanol, 2-(2-Butoxyethoxy)ethanol, 1-Methoxy-2-propanol, 3-Methoxypropanol oder Mischungen aus den genannten Alkoholen eingesetzt wird.

9. Verfahren nach einem der vorangegangenen Ansprüche, wobei die Carbonsäure und/oder das Carbonsäureanhydrid ausgewählt ist unter aromatischen Mono-, Di-, Tri- und Tetracarbonsäuren, aliphatischen Mono- und Dicarbonsäuren, Hydroxy-carbonsäuren, alicyclischen Mono-, Di-, Tri- und Tetracarbonsäuren, heterocyclischen Dicarbonsäuren, den Anhydriden der zuvor genannten Carbonsäuren und Mischungen davon.

10. Verfahren nach einem der vorangegangenen Ansprüche, wobei die Carbonsäure und/oder das Carbonsäureanhydrid ausgewählt ist unter Essigsäure, Essigsäureanhydrid, Benzoesäure, Benzoesäureanhydrid, Phthalsäure, Phthalsäureanhydrid, Isophthalsäure, Terephthalsäure, Trimellitsäure, Trimellitsäureanhydrid, Pyromellitsäure und Pyromellitsäuredianhydrid.

11. Verfahren nach einem der Ansprüche 1 bis 10 zur Herstellung von Bis(2-ethylhexyl)terephthalat durch Umsetzung von Terephthalsäure mit 2-Ethylhexanol.

12. Verfahren nach einem der Ansprüche 1 bis 10 zur Herstellung von 2-Butoxyethylacetat, 2-(2-Butoxyethoxy)-ethylacetat, 1-Methoxy-2-propylacetat und 3-Methoxypropylacetat durch Umsetzung von 2-Butoxyethanol, 2-(2-Butoxy-ethoxy)-ethanol, 1-Methoxy-2-propanol oder 3-Methoxypropanol mit Essigsäure oder Essigsäureanhydrid.

13. Verfahren nach einem der vorangegangenen Ansprüche, wobei der wenigstens eine Alkohol R¹-OH und/oder der wenigstens eine Alkohol R²-[O-X]ₙ-OH in einem 1,01- bis 2,0-fachen molaren Überschuss, bezogen auf die Carbonsäureäquivalente des eingesetzten Carbonsäurematerials, vorliegt.

14. Verfahren nach einem der vorangegangenen Ansprüche, wobei die eingesetzte Methansulfonsäure einen Gesamtchlorgehalt von höchstens 20 ppm, bevorzugt von höchstens 5 ppm, insbesondere von höchstens 1 ppm, aufweist.

15. Verfahren nach Anspruch 14, wobei die eingesetzte Methansulfonsäure einen Sulfatgehalt von höchstens 20 ppm aufweist.

16. Verfahren nach einem der vorangegangenen Ansprüche, wobei der Katalysator in einer Menge von 0,5 bis 5 mol%, bezogen auf die Anzahl der umzusetzenden Carbonsäuregruppen, eingesetzt wird.

## Claims

1. A process for the production of carboxylic esters, in which a reaction mixture which comprises at least one carboxylic acid and/or at least one carboxylic anhydride, and which comprises at least one alcohol R¹-OH and/or at least one alcohol R²-[O-X]ₙ-OH, in which
R¹ is selected among unbranched and branched C₅-C₁₃-alkyl moieties and C₅-C₆-cycloalkyl moieties, where the cycloalkyl moieties are unsubstituted or can be substituted by at least one C₁-C₁₀-alkyl moiety,
R² is selected from unbranched C₁-C₁₃-alkyl moieties and branched C₃-C₁₃-alkyl moieties,
X is an unbranched C₂-C₅-alkylene group or branched C₃-C₅-alkylene group, and
n has the value 1, 2 or 3,
is reacted in a reaction system composed of one or more reactors,
with the proviso that the reaction takes place
- in the presence of at least one catalyst selected from methanesulfonic acid, where the sulfate content of the methanesulfonic acid is at most 50 ppm,
- with introduction, into the reaction system, of a gas that is inert under the reaction conditions,
- at a reaction mixture temperature of from 125°C to 240°C, and
- with distillative removal, in the form of an azeotropic mixture with the alcohol R¹-OH and/or R²-[O-X]ₙ-OH used, of at least one portion of the water formed during the reaction,
where at least to some extent the alcohol R¹-OH and/or R²-[O-X]ₙ-OH removed by distillation is returned to the reaction system.

2. The process according to claim 1, where the inert gas is passed into at least one of the reactors below the liquid surface of the reaction mixture, and the inert gas bubbles through the reaction mixture.

3. The process according to any of the preceding claims, where the reaction system comprises a cascade of at least two reactors, and the inert gas is introduced at least into the first reactor of the cascade.

4. The process according to any of the preceding claims, where the reaction is carried out continuously.

5. The process according to any of the preceding claims, where the moiety R¹ is selected from n-octyl, 2-ethylhexyl, n-nonyl, isononyl, isodecyl, 2-propylheptyl, n-undecyl, isoundecyl.

6. The process according to any of the preceding claims, where 2-ethylhexanol is used as alcohol R¹-OH.

7. The process according to any of the preceding claims, where, in the formula R²-[O-X]ₙ-OH
R² is selected from unbranched C₁-C₉-alkyl moieties and branched C₃-C₉-alkyl moieties,
X is an unbranched C₂-C₃-alkylene group or a branched C₃-C₄-alkylene group, and
n has the value 1 or 2.

8. The process according to any of the preceding claims, where 2-butoxyethanol, 2-(2-butoxyethoxy)-ethanol, 1-methoxy-2-propanol, 3-methoxypropanol, or a mixture of the alcohols mentioned is used as alcohol R²-[O-X]ₙ-OH.

9. The process according to any of the preceding claims, where the carboxylic acid and/or the carboxylic anhydride is selected from aromatic mono-, di-, tri-, and tetracarboxylic acids, aliphatic mono- and dicarboxylic acids, hydroxycarboxylic acids, alicyclic mono-, di-, tri-, and tetracarboxylic acids, heterocyclic dicarboxylic acids, the anhydrides of the abovementioned carboxylic acids, and mixtures thereof.

10. The process according to any of the preceding claims, where the carboxylic acid and/or the carboxylic anhydride is selected from acetic acid, acetic anhydride, benzoic acid, benzoic anhydride, phthalic acid, phthalic anhydride, isophthalic acid, terephthalic acid, trimellitic acid, trimellitic anhydride, pyromellitic acid, and pyromellitic dianhydride.

11. The process according to any of claims 1 to 10 for the production of bis(2-ethylhexyl) terephthalate by reaction of terephthalic acid with 2-ethylhexanol.

12. The process according to any of claims 1 to 10 for the production of 2-butoxyethyl acetate, 2-(2-butoxy-ethoxy)ethyl acetate, 1-methoxy-2-propyl acetate and 3-methoxypropyl acetate via reaction of 2-butoxyethanol, 2-(2-butoxyethoxy)ethanol, 1-methoxy-2-propanol, or 3-methoxypropanol with acetic acid or acetic anhydride.

13. The process according to any of the preceding claims, where the at least one alcohol R¹-OH and/or the at least one alcohol R²-[O-X]ₙ-OH are/is present in a 1.01- to 2.0-fold molar excess, based on the carboxylic acid equivalents of the carboxylic acid material used.

14. The process according to any of the preceding claims, where the total chlorine content of the methanesulfonic acid used is at most 20 ppm, preferably at most 5 ppm, in particular at most 1 ppm.

15. The process according to Claim 14, where the sulfate content of the methanesulfonic acid used is at most 20 ppm.

16. The process according to any of the preceding claims, where the amount used of the catalyst, based on the number of the carboxylic acid groups to be reacted, is from 0.5 to 5 mol%.

## Revendications

1. Procédé pour la préparation d'esters carboxyliques, dans lequel on transforme un mélange réactionnel dans un système réactionnel constitué d'un ou plusieurs réacteurs, qui comprend au moins un acide carboxylique et/ou au moins un anhydride carboxylique et au moins un alcool R¹-OH et/ou au moins un alcool R²-[O-X]ₙ-OH, dans lequel
R¹ est choisi parmi des radicaux C₅₋₁₃-alkyle linéaires et ramifiés et des radicaux C₅₋₆-cycloalkyle, les radicaux cycloalkyle pouvant être non substitués ou substitués par au moins un radical C₁₋₁₀-alkyle,
R² est choisi parmi des radicaux C₁₋₁₃-alkyle linéaires et des radicaux C₃₋₁₃-alkyle ramifiés,
X représente un groupe C₂₋₅-alkylène linéaire ou un groupe C₃₋₅-alkylène ramifié et
n présente les valeurs de 1, 2 ou 3,
étant entendu que la transformation est effectuée
- en présence d'au moins un catalyseur, qui est choisi parmi l'acide méthanesulfonique, l'acide méthanesulfonique présentant une teneur en sulfates d'au plus 50 ppm,
- avec introduction dans le système réactionnel d'un gaz inerte dans les conditions de réaction,
- à une température du mélange réactionnel de 125°C jusqu'à 240°C et
- par la séparation par distillation d'au moins une partie de l'eau formée pendant la réaction sous forme d'un mélange azéotropique avec l'alcool utilisé R¹-OH et/ou R²-[O-X]ₙ-OH,
l'alcool distillé R¹-OH et/ou R²-[O-X]ₙ-OH étant au moins partiellement reconduit dans le système réactionnel.

2. Procédé selon la revendication 1, dans lequel on introduit le gaz inerte dans au moins un des réacteurs sous la surface liquide du mélange réactionnel et on fait buller le gaz inerte dans le mélange réactionnel.

3. Procédé selon l'une quelconque des revendications précédentes, dans lequel le système réactionnel comprend une cascade d'au moins deux réacteurs et on introduit le gaz inerte au moins au premier réacteur de la cascade.

4. Procédé selon l'une quelconque des revendications précédentes, dans lequel la transformation est mise en oeuvre dans une opération en continu.

5. Procédé selon l'une quelconque des revendications précédentes, dans lequel le radical R¹ est choisi parmi n-octyle, 2-éthylhexyle, n-nonyle, isononyle, isodécyle, 2-propylheptyle, n-undécyle, isoundécyle.

6. Procédé selon l'une quelconque des revendications précédentes, dans lequel le 2-éthylhexanol est utilisé en tant qu'alcool R¹-OH.

7. Procédé selon l'une quelconque des revendications précédentes, dans lequel dans la formule R²-[O-X]ₙ-OH
R² est choisi parmi des radicaux C₁₋₉-alkyle linéaires et des radicaux C₃₋₉-alkyle ramifiés,
X représente un groupe C₂₋₃-alkylène linéaire ou un groupe C₃₋₄-alkylène ramifié et
n présente les valeurs de 1 ou 2.

8. Procédé selon l'une quelconque des revendications précédentes, dans lequel le 2-butoxyéthanol, le 2-(2-butoxyéthoxy)éthanol, le 1-méthoxy-2-propanol, le 3-méthoxypropanol ou des mélanges des alcools mentionnés est/sont utilisé(s) en tant qu'alcool R²-[O-X]ₙ-OH.

9. Procédé selon l'une quelconque des revendications précédentes, dans lequel l'acide carboxylique et/ou l'anhydride carboxylique est choisi parmi les acides aromatiques monocarboxyliques, dicarboxyliques, tricarboxyliques et tétracarboxyliques, les acides aliphatiques monocarboxyliques et dicarboxyliques, les acides hydroxycarboxyliques, les acides alicycliques monocarboxyliques, dicarboxyliques, tricarboxyliques et tétracarboxyliques, les acides dicarboxyliques hétérocycliques, les anhydrides des acides carboxyliques précédemment mentionnés et les mélanges de ceux-ci.

10. Procédé selon l'une quelconque des revendications précédentes, dans lequel l'acide carboxylique et/ou l'anhydride carboxylique est choisi parmi l'acide acétique, l'anhydride acétique, l'acide benzoïque, l'anhydride benzoïque, l'acide phtalique, l'anhydride phtalique, l'acide isophtalique, l'acide téréphtalique, l'acide trimellitique, l'anhydride trimellitique, l'acide pyromellitique et le dianhydride pyromellitique.

11. Procédé selon l'une quelconque des revendications 1 à 10 pour la préparation de téréphtalate de bis(2-éthylhexyle) par la transformation de l'acide téréphtalique avec du 2-éthylhexanol.

12. Procédé selon l'une quelconque des revendications 1 à 10 pour la préparation d'acétate de 2-butoxyéthyle, d'acétate de 2-(2-butoxyéthoxy)éthyle, d'acétate de 1-méthoxy-2-propyle et d'acétate de 3-méthoxypropyle par la transformation de 2-butoxyéthanol, de 2-(2-butoxyéthoxy)éthanol, de 1-méthoxy-2-propanol ou de 3-méthoxypropanol avec de l'acide acétique ou de l'anhydride acétique.

13. Procédé selon l'une quelconque des revendications précédentes, dans lequel l'au moins un alcool R¹-OH et/ou l'au moins un alcool R²-[O-X]ₙ-OH est/sont présent(s) en un excédent molaire d'un facteur de 1,01 jusqu'à 2,0, par rapport aux équivalents en acide carboxylique de la matière acide carboxylique utilisée.

14. Procédé selon l'une quelconque des revendications précédentes, dans lequel l'acide méthanesulfonique utilisé présente une teneur totale en chlore d'au plus 20 ppm, préférablement d'au plus 5 ppm, en particulier d'au plus 1 ppm.

15. Procédé selon la revendication 14, dans lequel l'acide méthanesulfonique utilisé présente une teneur en sulfates d'au plus 20 ppm.

16. Procédé selon l'une quelconque des revendications précédentes, dans lequel le catalyseur est utilisé en une quantité de 0,5 à 5% en moles, par rapport au nombre de groupes acides carboxyliques qui sont à transformer.
